(19) Europäisches Patentamt
European Patent Office
Office européen des brevets

(11) EP 1 666 142 A1

(12) EUROPEAN PATENT APPLICATION

(43) Date of publication:
**07.06.2006 Bulletin 2006/23**

(21) Application number: **05257253.4**

(22) Date of filing: **25.11.2005**

(51) Int Cl.:
***B01J 23/00*** $^{(2006.01)}$ ***B01J 27/057*** $^{(2006.01)}$
***B01J 37/02*** $^{(2006.01)}$ ***C07C 51/215*** $^{(2006.01)}$
***C07C 57/04*** $^{(2006.01)}$ ***C07C 253/24*** $^{(2006.01)}$
***C07C 255/08*** $^{(2006.01)}$

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **06.12.2004 US 633677 P**

(71) Applicant: **Rohm and Haas Company**
**Philadelphia, PA 19106-2399 (US)**

(72) Inventors:
- **Gaffney, Anne Mae**
  **West Chester,**
  **Pennsylvania 19380 (US)**
- **Gleaves, John T.**
  **Foley,**
  **Missouri 63347 (US)**
- **Han, Scott**
  **Lawrenceville,**
  **New Jersey 08648 (US)**
- **Song, Ruozhi**
  **Wilmington,**
  **Delaware 19809 (US)**

(74) Representative: **Kent, Venetia Katherine et al**
**Rohm and Haas (UK) Ltd.**
**European Patent Department**
**4th Floor, 22 Tudor Street**
**London EC4Y 0AY (GB)**

(54) **(Amm)oxidation catalyst and catalytic (amm)oxidation process for conversion of lower alkanes**

(57) The present invention relates to mixed metal oxide (MMO1) catalysts. MMO1 catalyst performance is improved with a sub-monolayer deposition of Te onto its surface by vapor deposition. The selectivity to acrylic acid improved by approximately 6% and the acrylic acid yield by 3%, absolute. Applying a similar Te loading onto MMO1 by wet impregnation methods did not improve catalytic performance. Post treatment of the Te vapor deposited MMO1 catalyst with oxygen at elevated temperatures gave improved catalytic performance when compared to a corresponding sample treated with an inert gas at the same elevated temperatures.

EP 1 666 142 A1

## Description

The present invention relates to a vapor deposition process for preparing improved (amm) oxidation catalysts. The present invention also relates to an improved single-step catalytic vapor phase (amm)oxidation process for the conversion of one or more $C_2$-$C_8$ alkanes to one or more oxidation products, including unsaturated carboxylic acids and unsaturated nitriles, whereby a higher yield of the oxidation products is achieved.

Unsaturated carboxylic acids such as acrylic acid and methacrylic acid are industrially important as starting materials for various synthetic resins, coating materials and plasticizers. Nitriles, such as acrylonitrile and methacrylonitrile, are industrially important intermediates for the preparation of fibers, synthetic resins, synthetic rubbers, and the like. Such unsaturated carboxylic acids and nitriles can be produced by catalytic (amm)oxidation of lower (i.e., $C_2$-$C_8$) alkanes and alkenes, such as ethane, ethane, propane, propene, butane (including n- and iso-butane), butane (including n- and iso-butene) and pentane (including n- and iso-pentane) and pentane (including n- and iso-pentene).

For example, the currently practiced commercial process for the production of acrylic acid involves a two-step catalytic vapor phase oxidation reaction using an alkene, propene, as the hydrocarbon starting material. In the two-step oxidation reaction, propene is converted to acrolein over a suitable mixed metal oxide catalyst in the first step. In the second step, acrolein product from the first step is converted to acrylic acid using a second suitable mixed metal oxide catalyst. In most cases, the catalyst formulations are proprietary to the catalyst supplier, but the technology is well established. Furthermore, it is known to include additional starting materials, including additional reactants, such as molecular oxygen and/or steam, and inert materials, such as nitrogen and carbon dioxide, along with the hydrocarbon starting material that is fed to such two-step oxidation processes. See, for example, U.S. Patent No. 5,218,146, which discloses a two-step catalytic vapor phase oxidation process for conversion of propene to acrylic acid. In the disclosure of U.S. Patent No. 5,218,146, carbon dioxide is fed to the two-step oxidation process in an amount of from 3 % to 50 % by volume, based upon the total volume of the starting materials, which also include propene and molecular oxygen. There is, however, no correlation provided, expressly or implicitly, in U.S. Patent No. 5,218,146 between the amount of carbon dioxide which is fed to the process and the yield of acrylic acid product.

The most popular method for producing nitriles is to subject an alkene (olefin), such as propene or isobutene, to a catalytic reaction with ammonia and oxygen in the presence of a suitable catalyst in a gaseous phase at a high temperature. There are various known catalysts suitable for conducting this reaction and, while many of the catalyst formulations are proprietary to the catalyst supplier, this technology is also well established. Furthermore, it is known to include additional starting materials, including additional reactants, such as molecular oxygen and/or steam, and inert materials, such as nitrogen and carbon dioxide, along with the hydrocarbon and ammonia starting materials that are fed to such two-step ammoxidation processes.

In view of the lower price of alkanes (for example, propane and isobutene) in comparison to alkenes (for example, propene and isobutene), attention has been drawn to the development of catalysts and processes for the production of unsaturated carboxylic acids and unsaturated nitriles in a single-step vapor phase (amm)oxidation process using the cheaper alkane as the hydrocarbon starting material. For example, catalysts capable of catalyzing the single-step oxidation of propane to acrylic acid in yields up to 52% have been developed and continue to be improved.

In addition, some refinements to the single-step oxidation process itself have been developed and further improvements to the single-step oxidation process continue to be sought and welcomed by industry. For example, it is known to include additional starting materials, including additional reactants, such as molecular oxygen and/or steam, as well as inert materials, such as nitrogen and carbon dioxide to act as diluents or heat moderators, along with the hydrocarbon starting material that is fed to the one-step oxidation process.

For example, U.S. Patent No. 6,646,158 which states that carbon dioxide may be fed to the oxidation process in amounts greater than 5 % by volume, based on the total volume of the feed gases, but no examples are provided that include feeding carbon dioxide to the disclosed process. Thus, carbon dioxide is not required for this process and no conclusions may be drawn from U.S. Patent No. 6,646,158 regarding the efficacy of carbon dioxide as a diluent or heat moderator. In addition, U.S. Patent No. 6,693,059 discloses the possibility of feeding a diluting gas, such as nitrogen, argon, helium or carbon dioxide, in an amount of from 0 % to 20 %, by volume, to a single-step oxidation process which converts propane to acrylic acid. This patent, however, is focused on the catalyst composition and activity and no examples are provided that include feeding carbon dioxide to the single-step oxidation process. O.V. Krylov et al., in "*The regularities in the interaction of alkanes with $CO_2$ on oxide catalysts*," Catalysis Today 24 (1995) 371-375, disclose the use of carbon dioxide as a non-traditional oxidant in the oxidation of methane, ethane and propane, but the products include only synthesis gases (hydrogen and carbon monoxide) and simple oxydehydrogenation products such as alkenes, without production of unsaturated carboxylic acids or nitriles. Thus, none of these prior disclosures explore or discuss the use of carbon dioxide as a feed component to single-step (amm)oxidation processes for increasing the production of (amm)oxidation products, including unsaturated carboxylic acids and nitriles.

Thus, the chemical industry would welcome further improvements to increase the yields of single-step (amm) oxidation processes for the conversion of one or more $C_2$ to $C_8$ alkanes to valuable (amm)oxidation products, including

unsaturated carboxylic acids and nitriles.

Inventors have unexpectedly discovered that the MMO1 catalyst performance is improved with a sub-monolayer deposition of Te onto its surface by vapor deposition. The selectivity to acrylic acid improved by approximately 6% and the acrylic acid yield by 3%, absolute. Applying a similar Te loading onto MMO1 by wet impregnation methods did not improve catalytic performance. Post treatment of the Te vapor deposited MMO1 catalyst with oxygen at elevated temperatures gave improved catalytic performance when compared to a corresponding sample treated with an inert gas at the same elevated temperatures.

Accordingly, the invention provides an improved (amm)oxidation catalyst comprising: one or more modified mixed metal oxide catalysts having the empirical formula:

$$M_eMoV_aNb_bX_cZ_dO_n$$

wherein $M_e$ is at least one or more chemical modifying agents, X is at least one element selected from the group consisting of Te and Sb, Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements, $0.1 \le a \le 1.0$, $0.01 \le b \le 1.0$, $0.01 \le c \le 1.0$, $0 \le d \le 1.0$ and n, e are determined by the oxidation states of the other elements; wherein the catalyst is enhanced in X and Z by vapor depositing at least element of X, Z or combinations thereof; and wherein the modified catalyst exhibits improved catalyst performance characteristics selected from the group consisting of: optimized catalyst properties, yields of oxygenates including unsaturated carboxylic acids, from their corresponding alkanes, alkenes or combinations of corresponding alkanes and alkenes at constant alkane/alkene conversion, selectivity of oxygenate products, including unsaturated carboxylic acids, from their corresponding alkanes, alkenes or combinations of corresponding alkanes and alkenes, optimized feed conversion, cumulative yield of the desired oxidation product, and combinations thereof, as compared to the unmodified catalyst.

Accordingly, the invention also provides a process for preparing an improved (amm)oxidation catalyst comprising the step of: depositing one or more elements X and Z in the vapor phase, wherein X is at least one element selected from the group consisting of Te and Sb, Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements, to one or more metals to one or more mixed metal catalysts.

Accordingly, the invention also provides a surface modified (amm)oxidation catalyst comprising: one or more modified mixed metal oxide catalysts having the empirical formula:

$$M_eMoV_aNb_bX_cZ_dO_n$$

wherein $M_e$ is at least one or more chemical modifying agents, X is at least one element selected from the group consisting of Te and Sb, Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements, $0.1 \le a \le 1.0$, $0.01 \le b \le 1.0$, $0.01 \le c \le 1.0$, $0 \le d \le 1.0$ and n, e are determined by the oxidation states of the other elements; wherein the catalyst surface is modified in X and Z by vapor depositing at least element of X, Z or combinations thereof on to the surface of the mixed metal oxide catalyst; and wherein the surface modified catalyst exhibits improved catalyst performance characteristics selected from the group consisting of: optimized catalyst properties, yields of oxygenates including unsaturated carboxylic acids, from their corresponding alkanes, alkenes or combinations of corresponding alkanes and alkenes at constant alkane/alkene conversion, selectivity of oxygenate products, including unsaturated carboxylic acids, from their corresponding alkanes, alkenes or combinations of corresponding alkanes and alkenes, optimized feed conversion, cumulative yield of the desired oxidation product, and combinations thereof, as compared to the unmodified catalyst.

Accordingly, the invention also provides a process for modifiying the surface of one or more mixed metal oxide catalysts comprising the step of: depositing one or more elements X and Z in the vapor phase, wherein X is at least one element selected from the group consisting of Te and Sb, Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements, to one or more metals to one or more mixed metal catalysts.

Accordingly, the invention also provides a modified catalyst system comprising two or more layers: a first catalyst layer comprising one or more modified mixed metal oxide catalysts and (b) at least a second catalyst layer comprising at least one unmodified or modified metal oxide, supported or unsupported, and is oriented downstream from the first catalyst layer; wherein the catalyst is enhanced in X and Z by vapor depositing at least element of X, Z or combinations thereof.

Accordingly, the invention also provides a surface modified catalyst system comprising two or more layers: a first catalyst layer comprising one or more modified mixed metal oxide catalysts and (b) at least a second catalyst layer comprising at least one unmodified or modified metal oxide, supported or unsupported, and is oriented downstream

from the first catalyst layer; wherein the catalyst surface is modified in X and Z by vapor depositing at least element of X, Z or combinations thereof on to the surface of the mixed metal oxide catalyst.

Accordingly, the invention also provides a process for enhancing, rebuilding, replenishing or reconstructing the surface of one or more mixed metal oxide catalysts comprising the step of: depositing one or more elements X and Z in the vapor phase, wherein X is at least one element selected from the group consisting of Te and Sb, Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements, to one or more metals to one or more mixed metal catalysts.

Vapor deposition onto the mixed metal oxide catalyst surface in accordance with the invention serves as a method of preparing improved (amm)oxidation catalysts, modified (amm)oxidation catalysts and a method for improving the performance of an on-stream catalyst that may have undergone performance degradation.

Vapor deposition is accomplished in accordance with the invention by techniques known in the art, including physical vapor deposition, chemical vapor deposition, sputtering, anodic or cathodic arc deposition, thermal or plasma-supported gas phase deposition, and the like.

Regarding the conversion of propane to acrylic acid, synthesis of a mixed metal oxides with catalytic performances that match or out perform catalysts with compositions falling within the claims in the patent art may be realized by vapor deposition.

According to one embodiment, a Mo-V-Ox mixed metal oxide is synthesized by conventional methods (e.g., hydrothermal, spray dry, evaporative methods, etc.) and then treated by vapor deposition to provide monolayer coverage of Te and Nb so that the bulk compositional levels fall far below the patent claims of MMO1. Assuming the catalyst performance is largely based upon its surface chemistry, competitive acrylic acid yields are realized with catalysts prepared by these methods.

As used herein, the term "surface modified catalyst" which is equivalent to "surface treated catalysts" which is also equivalent to "post-treated catalysts" refers to any chemical, physical and combinations of chemical and physical modification or modifications of the surface layer, or initial layers of a multilayered catalyst, of one or more prepared catalysts as compared to corresponding catalysts having undergone no such surface modification or surface modifications (also referred to as unmodified catalysts, equivalently referred to as untreated catalysts). As used herein, the term "modified catalyst" which is equivalent to "treated catalysts" which is also equivalent to "post-treated catalysts" refers to any chemical, physical and combinations of chemical and physical modification or modifications of one or more prepared catalysts as compared to corresponding catalysts having undergone no such modification or modifications (also referred to as unmodified catalysts, equivalently referred to as untreated catalysts). Modifications to prepared catalysts include, but are not limited to, any differences in the modified catalysts as compared to corresponding unmodified catalysts. Suitable modifications to catalysts include, for example, structural changes, spectral changes (including position and intensity of characteristic X-ray diffraction lines, peaks and patterns), spectroscopic changes, chemical changes, physical changes, compositional changes, changes in physical properties, changes in catalytic properties, changes in performance characteristics in conversions of organic molecules, changes in yields of organic products from corresponding reactants, changes in catalyst activity, changes in catalyst selectivity and combinations thereof. This includes one or more chemical modifying agents (e.g. a reducing agent such as an amine), one or more physical processes (e.g. mechanical grinding at cryogenic temperatures also referred to as "cryo-grinding") and combinations of one or more chemical modifying agents and one or more physical processes. The term "cryo" in front of any treatment term refers to any treatment that occurs with cooling, under freezing temperatures, at cryogenic temperatures and any use of cryogenic fluids. Suitable cryogenic fluids include, but are not limited to for example, any conventional cryogens and other coolants such as chilled water, ice, compressible organic solvents such as freons, liquid carbon dioxide, liquid nitrogen, liquid helium and combinations thereof. Suitable chemical and physical modification of prepared (untreated) catalysts results in unexpected improvements in treated catalyst efficiency and selectivity in alkane, alkene or alkane and alkene oxidations as compared to corresponding untreated catalysts and improved yields of oxygenated products using modified catalysts using modified catalysts as compared to unmodified catalysts. The term prepared catalysts refers to unmodified catalysts. The prepared catalysts are obtained from commercial sources or are prepared by conventional preparative methods, including methods described herein. The term "treated catalysts" and "modified catalysts" does not refer to or include regenerated, reconditioned and recycled catalysts. The term conditioning refers to conventional heating of prepared metal oxide catalysts with gases including hydrogen, nitrogen, oxygen and selected combinations thereof.

As used herein, the term "cumulatively converting" refers producing a desired product stream from one or more specific reactants using one or more modified catalysts and modified catalyst systems of the invention under specific reaction conditions. As an illustrative embodiment, cumulatively converting an alkane to its corresponding unsaturated carboxylic acid means that the modified catalyst(s) utilized will produce a product stream comprising the unsaturated carboxylic acid corresponding to the added alkane when acting on a feed stream(s) comprising the alkane and molecular oxygen under the designated reaction conditions. According to a separate embodiment, the invention also provides a process for optimizing recycle conversion of specific alkanes, alkenes, alkanes and alkenes and their corresponding

oxygenate products.

As used herein, mixed metal oxide catalyst refers to a catalyst comprising more than one metal oxide. The term "catalytic system" refers to two or more catalysts. For example, platinum metal and indium oxide impregnated on an alumina support defines both a catalytic system and a mixed metal oxide catalyst. Yet another example of both is palladium metal, vanadium oxide and magnesium oxide impregnated on silica.

Any one or more metal oxide catalysts are usefully modified and utilized in catalytic conversions of molecules containing carbon in accordance with the invention. According to one embodiment, the modified catalysts are modified mixed metal oxide catalysts useful for catalytically converting alkanes, alkenes and combinations of alkanes and alkenes to their corresponding oxygenates. The prepared metal oxide catalysts are modified using the one or more chemical, physical and combined chemical and physical treatments to provide modified metal oxide catalysts, including modified mixed metal oxide catalysts.

According to one embodiment of the invention, suitable prepared catalysts used and modified in accordance with the invention are one or more mixed metal oxide catalysts having a catalyst having the empirical formula

$$MoV_aNb_bX_cZ_dO_n$$

wherein X is at least one element selected from the group consisting of Te and Sb, Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements, $0.1 \le a \le 1.0$, $0.01 \le b \le 1.0$, $0.01 \le c \le 1.0$, $0 \le d \le 1.0$ and n is determined by the oxidation states of the other elements. Preparation of the mixed metal oxide (MMO) catalysts is described in U. S. Patent Nos. 6,383,978; 6,641,996; 6,518,216; 6,403,525; 6,407,031; 6,407,280; and 6,589,907; U. S. Publication Application No. 20030004379; U. S. Provisional Application Ser. Nos. 60/235,977; 60/235,979; 60/235,981; 60/235,984; 60/235,983; 60/236,000; 60/236,073; 60/236,129; 60/236,143; 60/236,605; 60/236,250; 60/236,260; 60/236,262; 60/236,263; 60/283,245; and 60/286,218; and EP Patent Nos. EP 1 080 784; EP 1 192 982; EP 1 192 983; EP 1 192 984; EP 1 192 986; EP 1 192 987; EP 1 192 988; EP 1 192 982; EP 1 249 274; and EP 1 270 068. The synthesis of such MMO (mixed metal oxide) catalysts is accomplished by several methods well known by those having skill in the art. A precursor slurry of mixed metal salts is first prepared by conventional methods and methods described above that include, but are not limited to for example, rotary evaporation, drying under reduced pressure, hydrothermal methods, coprecipitation, solid-state synthesis, impregnation, incipient wetness, sol gel processing and combinations thereof. After the precursor slurry is prepared it is dried according to conventional drying methods including, but not limited to for example, drying in ovens, spray drying and freeze drying. The dried precursor is then calcined to obtain prepared MMO catalysts using well known techniques and techniques described above to those having skill in the art including, but not limited to for example, flow calcinations, static calcinations, rotary calcinations and fluid-bed calcinations. In some cases the prepared MMO catalysts are further milled to improve their catalytic activity.

It is noted that promoted mixed metal oxides having the empirical formulae $Mo_jV_mTe_nNb_yZ_zO_o$ or $W_jV_mTe_n$-$Nb_yZ_zO_o$, wherein Z, j, m, n, y, z and o are as previously defined, are particularly suitable for use in connection with the present invention. Additional suitable embodiments are either of the aforesaid empirical formulae, wherein Z is Pd. Suitable solvents for the precursor solution include water; alcohols including , but not limited to, methanol, ethanol, propanol, and diols, etc.; as well as other polar solvents known in the art. Generally, water is preferred. The water is any water suitable for use in chemical syntheses including, without limitation, distilled water and de-ionized water. The amount of water present is preferably an amount sufficient to keep the elements substantially in solution long enough to avoid or minimize compositional and/or phase segregation during the preparation steps. Accordingly, the amount of water will vary according to the amounts and solubilities of the materials combined. Preferably, though lower concentrations of water are possible for forming a slurry, as stated above, the amount of water is sufficient to ensure an aqueous solution is formed, at the time of mixing.

According to a separate embodiment of the invention, suitable prepared mixed metal oxide catalysts used and modified in accordance with the invention are one or more promoted mixed metal oxide catalysts having the empirical formula

$$J_jM_mN_nY_yZ_zO_o$$

wherein J is at least one element selected from the group consisting of Mo and W, M is at least one element selected from the group consisting of V and Ce, N is at least one element selected from the group consisting of Te, Sb and Se, Y is at least one element selected from the group consisting of Nb, Ta, Ti, Al, Zr, Cr, Mn, Fe, Ru, Co, Rh, Ni, Pt, Sb, Bi, B, In, As, Ge, Sn, Li, Na, K, Rb, Cs, Fr, Be, Mg, Ca, Sr, Ba, Ra, Hf, Pb, P, Pm, Eu, Gd, Dy, Ho, Er, Tm, Yb and Lu, and Z is selected from the group consisting of Ni, Pd, Cu, Ag and Au; and wherein, when j = 1, m = 0.01 to 1.0, n = 0.01 to 1.0, y = 0.01 to 1.0, z = 0.001 to 0.1 and o is dependent on the oxidation state of the other elements. Preparation of the mixed metal catalysts is described in U. S. Patent Nos. 6,383,978; 6,641,996; 6,518,216; 6,403,525; 6,407,031;

6,407,280; and 6,589,907; U. S. Provisional Application Ser. Nos. 60/235,977; 60/235,979; 60/235,981; 60/235,984; 60/235,983; 60/236,000; 60/236,073; 60/236,129; 60/236,143; 60/236,605; 60/236,250; 60/236,260; 60/236,262; 60/236,263; 60/283,245; and 60/286,218; and EP Patent Nos. EP 1 080 784; EP 1 192 982; EP 1 192 983; EP 1 192 984; EP 1 192 986; EP 1 192 987; EP 1 192 988; EP 1 192 982; and EP 1 249 274.

According to a separate embodiment of the invention, suitable prepared catalysts modified and used in accordance with the invention are one or more mixed metal oxide catalysts having the empirical formula

$$A_aD_bE_cX_dO_e$$

wherein A is at least one element selected from the group consisting of Mo and W, D is at least one element selected from the group consisting of V and Ce, E is at least one element selected from the group consisting of Te, Sb and Se, and X is at least one element selected from the group consisting of Nb, Ta, Ti, Al, Zr, Cr, Mn, Fe, Ru, Co, Rh, Ni, Pt, Sb, Bi, B, In, As, Ge, Sn, Li, Na, K, Rb, Cs, Fr, Be, Mg, Ca, Sr, Ba, Hf, Pb, P, Pm, Eu, Gd, Dy, Ho, Er, Tm, Yb and Lu; and a = 1, b = 0.01 to 1.0, c = 0.01 to 1.0, d = 0.01 to 1.0, and e is dependent on the oxidation state of the other elements. The catalyst composition is treated to exhibit peaks at X-ray diffraction angles (2θ) of 22.1°, 27.1°, 28.2°, 36.2°, 45.2°, and 50.0°, with a relative increase in a diffraction peak at the diffraction angle (2θ) of 27.1 degrees when compared with an untreated catalyst of like empirical formula.

In this regard, in addition to the above noted peak at 27.1 degrees, the preferred mixed metal oxide exhibits the following five main diffraction peaks at specific diffraction angles (2θ) in the X-ray diffraction pattern of the treated mixed metal oxide (as measured using Cu-Kα radiation as the source):

| | X-ray lattice plane | |
|---|---|---|
| Diffraction angle 2θ (±0.3°) | Spacing medium (Å) | Relative intensity |
| 22.1° | 4.02 | 100 |
| 28.2° | 3.16 | 20~150 |
| 36.2° | 2.48 | 5~60 |
| 45.2° | 2.00 | 2~40 |
| 50.0° | 1.82 | 2~40 |

The intensity of the X-ray diffraction peaks may vary upon the measuring of each crystal. However, the intensity, relative to the peak intensity at 22.1° being 100, is usually within the above ranges. Generally, the peak intensities at 2θ = 22.1° and 28.2° are distinctly observed. However, so long as the above five diffraction peaks are observable, the basic crystal structure is the same even if other peaks are observed in addition to the five diffraction peaks (e.g. at 27.1 degrees), and such a structure is useful for the present invention. Preparation of the mixed metal catalysts is described in U. S. Patent Application Publication No. 20020183547 and European Patent Publication No. EP 1 249 274.

Other suitable prepared catalysts modified using the invention include those described in U.S. Patent No. 5,380,933 discloses a method for producing an unsaturated carboxylic acid comprising subjecting an alkane to a vapor phase catalytic oxidation reaction in the presence of a catalyst containing a mixed metal oxide comprising, as essential components, Mo, V, Te, O and X, wherein X is at least one element selected from the group consisting of niobium, tantalum, tungsten, titanium, aluminum, zirconium, chromium, manganese, iron, ruthenium, cobalt, rhodium, nickel, palladium, platinum, antimony, bismuth, boron, indium and cerium; and wherein the proportions of the respective essential components, based on the total amount of the essential components, exclusive of oxygen, satisfy the following relationships: 0.25 < r(Mo) < 0.98, 0.003 < r(V) < 0.5, 0.003 < r(Te) < 0.5 and 0.003 < r(X) < 0.5, wherein r(Mo), r(V), r(Te) and r(X) are the molar fractions of Mo, V, Te and X, respectively, based on the total amount of the essential components exclusive of oxygen.

Yet other suitable examples of prepared catalysts modified using the invention include those described in Published International Application No. WO 00/29106 discloses a catalyst for selective oxidation of propane to oxygenated products including acrylic acid, acrolein and acetic acid, said catalyst system containing a catalyst composition comprising

$$Mo_aV_bGa_cPd_dNb_eX_f$$

wherein X is at least one element selected from La, Te, Ge, Zn, Si, In and W,
a is 1,
b is 0.01 to 0.9,
c is >0 to 0.2,
d is 0.0000001 to 0.2,

e is >0 to 0.2, and
f is .0 to 0.5; and
wherein the numerical values of a, b, c, d, e and f represent the relative gram-atom ratios of the elements Mo, V, Ga, Pd, Nb and X, respectively, in the catalyst and the elements are present in combination with oxygen.

Yet other suitable examples of prepared catalysts modified using the invention include those described in Japanese Laid-Open Patent Application Publication No. 2000-037623 and European Published Patent Application No. 0 630 879 B1. Other suitable catalysts for a variety of vapor phase oxidation reactions are described fully in U.S. Patent Nos. 6,383,978, 6,403,525, 6,407,031, 6,407,280, 6,461,996, 6,472,552, 6,504,053, 6,589,907 and 6,624,111.

By way of an illustrative example, when a mixed metal oxide of the formula $Mo_aV_bTe_cNb_dO_e$ (wherein the element A is Mo, the element D is V, the element E is Te and the element X is Nb) is to be prepared, an aqueous solution of niobium oxalate and a solution of aqueous nitric acid may be added to an aqueous solution or slurry of ammonium heptamolybdate, ammonium metavanadate and telluric acid, so that the atomic ratio of the respective metal elements would be in the prescribed proportions. In one specific illustration, it is further contemplated that a 5% aqueous nitric acid is mixed with niobium oxalate solution in a ratio of 1:10 to 1.25:1 parts by volume acid solution to oxalate solution, and more preferably 1:5 to 1:1 parts by volume acid solution to oxalate solution.

For example, when a promoted mixed metal oxide of the formula $Mo_jV_mTe_nNb_yAu_zO_f$ wherein the element J is Mo, the element M is V, the element N is Te, the element Y is Nb, and the element Z is Au, is to be prepared, an aqueous solution of niobium oxalate may be added to an aqueous solution or slurry of ammonium heptamolybdate, ammonium metavanadate, telluric acid and ammonium tetrachloroaurate, so that the atomic ratio of the respective metal elements would be in the prescribed proportions.

A unmodified mixed metal oxide (promoted or not), thus obtained, exhibits excellent catalytic activities by itself. However, the unmodified mixed metal oxide is converted to a catalyst having higher activities by one or more chemical, physical and combinations of chemical and physical treatments.

Modified metal oxide catalysts are obtained by treating chemical, physical and combinations of chemical and physical treatments of suitable prepared metal oxide catalyst. Optionally, the modified catalysts are further modified by conventional processing techniques well known to persons having skill in this art.

It was discovered that the MMO1 catalyst performance is improved with a sub-monolayer deposition of Te onto its surface by vapor deposition. The selectivity to acrylic acid improved by approximately 6% and the acrylic acid yield by 3%, absolute. Applying a similar Te loading onto MMO1 by wet impregnation methods did not improve catalytic performance. Post treatment of the Te vapor deposited MMO1 catalyst with oxygen at elevated temperatures gave improved catalytic performance when compared to a corresponding sample treated with an inert gas at the same elevated temperatures.

Further improvements in catalyst performance are anticipated with the optimization of the Te vapor deposition loading level on the mixed metal oxide surface, optimization of the oxygen thermal post treatment, evaluation of other metals (and related compounds) and combination of metals (and related compounds) added by vapor deposition to mixed metal oxide surfaces, including an optimized oxygen thermal post treatment.

Vapor deposition onto the mixed metal oxide catalyst surface serves as a means of improving the performance of an on-stream catalyst that may have undergone performance degradation.

Vapor deposition may be accomplished by techniques known in the art, including physical vapor deposition, chemical vapor deposition, sputtering, anodic or cathodic arc deposition, thermal or plasma-supported gas phase deposition, and the like.

Regarding the conversion of propane to acrylic acid, synthesis of a mixed metal oxides with catalytic performances that match or out perform catalysts with compositions falling within the claims in the patent art may be realized by vapor deposition. For example, one may propose that a Mo-V-Ox mixed metal oxide may be synthesized by conventional methods (e.g., hydrothermal, spray dry, evaporative methods, etc.) and then treated by vapor deposition to provide monolayer coverage of Te and Nb so that the bulk compositional levels fall far below the patent claims of MMO1. Assuming the catalyst performance is largely based upon its surface chemistry, the hypothesis is that competitive acrylic acid yields may be realized with catalysts prepared by these methods.

A number of key discoveries and disclosures in accordance with the present invention include, but are not limited to for example, the following: MMO1 samples modified by atomic beam deposition are more selective for the production of acrylic acid. The MMO1 sample promoted with 0.1% monolayer of Te showed the highest selectivity (an increase of between 5 - 8% over the non-promoted sample). In TAP (temporal analysis of products) vacuum pulse response experiments propene and acrolein (in approximately equal amounts) are the principal selective products when water is not present in the feed. Pulsing water produces acrylic acid, and decreases acrolein production. If propene instead of propane is pulsed over the same MMO1 catalyst at the same pulse intensity and reaction conditions 5 times less acrolein is produced. Over an oxidized catalyst propene mainly produces $CO_2$, and propane provides selective products. Water desorbs more slowly from MMO1 samples modified by atomic beam deposition of tellurium. The addition of tellurium decreases catalyst activity under steady flow conditions. An apparent activation energy of 19 kcal/mol was

obtained for propane activation under vacuum pulse conditions. Comparison with values obtained in steady-state experiments indicates that the activation energy varies inversely with the catalyst oxidation state. Results of atmospheric pressure step transient experiments indicate the presence of an acrolein intermediate during the step input. The acrolein is converted to acrylic acid as water production increases. The selectivity is a function of the oxidation state of the catalyst surface, the gas phase composition, and contact time. The best performance is obtained when the catalyst is maintained in the optimum oxidation state. TAP experiments show that the optimum oxidation state for propane is different than for propene, and that when the catalyst is maintained in a higher oxidation state propane can be converted to acrylic acid without propene desorption. A general kinetic model was developed using the combined pulse response, step response and steady-state data. Step transient experiments indicate that higher conversion and yield can be obtained under non steady-state reaction conditions. Relative decreases in conversion and yield of as high as 50% was observed in going from nonsteady-state to steady-state conditions. Steady-state experiments comparing samples with different Te loadings indicate that the Te-loading that gives the highest performance is in the 0.1 - 0.2% range. TAP pulse response studies of MMO1 samples modified by atomic beam deposition, and by wet impregnation indicate that water desorbs more slowly from the sample containing 0.1% Te prepared by atomic beam deposition.

One kinetic model for propane conversion on MMO catalyst systems is the following:

Propane oxidation to acrylic acid can occur at a single site (K) or may involve desorption of an intermediate, which is then oxidized at a different site. Single site oxidation occurs if sufficient oxygen is available at the original propane adsorption site.

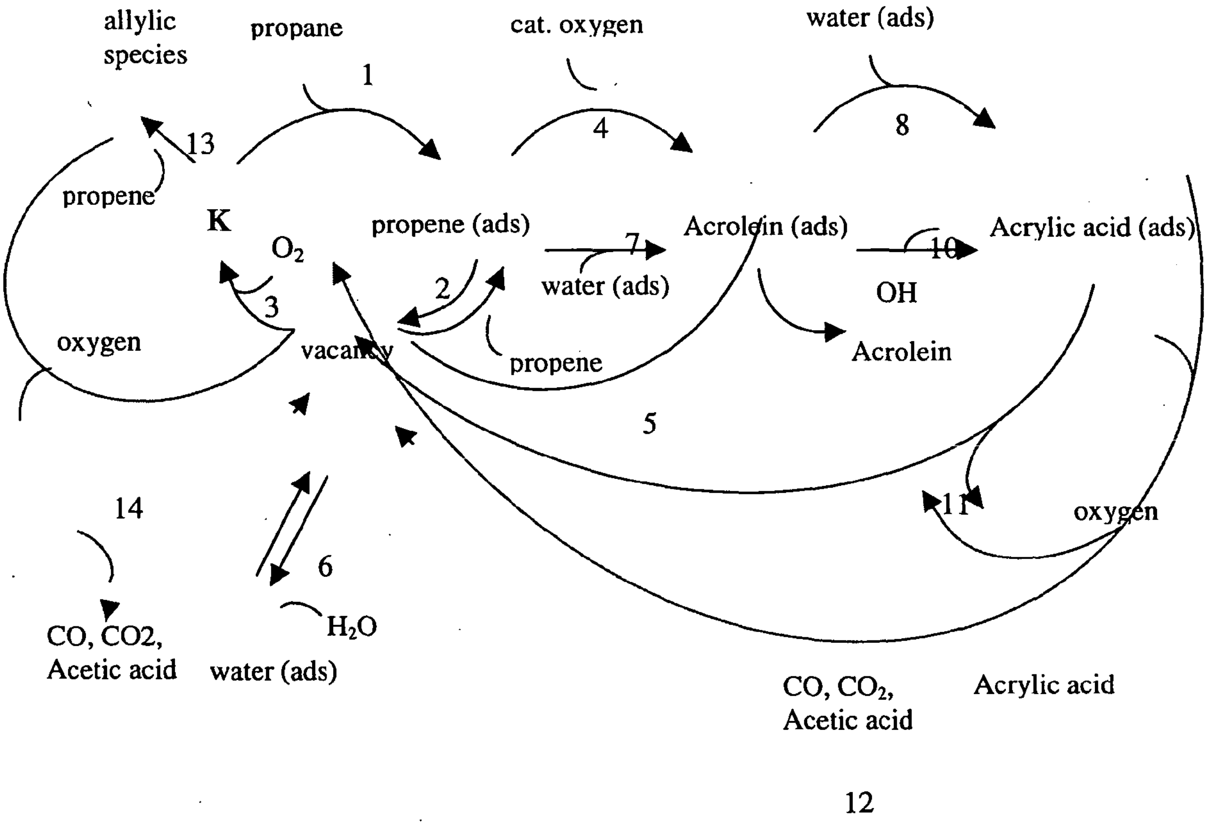


Assuming the kinetic model presented above, the efficiency of the process can be characterized using the relative yield, $\gamma$, (ratio of rates of acrylic acid and $CO_2$ production) given by:

$$\gamma R_{AA}/R_{CO2},$$

The relative yield for steady-state operation is be determined using graph theory. In the case of our steady-state experiments, acrolein is not observed, and it is not included in the following determination of the relative yield.

If the allylic route (steps 13, 14, 3) is neglected for $CO_2$ production, then $CO_2$ would be primarily produced in step 12, and it can be shown that the relative yield is given in Equation (1):

$$\gamma R_{AA}/R_{CO2} = k_{11} / k_{12} \qquad (1)$$

In this case $\gamma$ is an exponential function of temperature, does not depend on the gas composition, and can be viewed as the intrinsic yield. We can assume that the activation energy of step 12 ($CO_2$ production) is higher than the activation energy of step 11 (acrylic acid production), and the relative yield will decrease with temperature.

If the allylic route is not neglected, then $\gamma$ is a function of the gas composition, and will always be smaller than the value determined by equation (1). Thus, the ratio $k_{11}/k_{12}$ can be treated as an upper limit of the yield for the steady-state process. It can be shown that if the allylic route is present, the relative yield is given by:

$$\gamma = (k_{11} / k_{12})(1/(1+\alpha(C))(1/(1+\beta(C)), \qquad (2)$$

where $\alpha$ is a complex term that reflects the influence of the gas composition and $\beta$ is a complex term that reflects the influence of the catalyst oxidation state, and the water surface concentration. In this case, it is clear that the allylic route decreases performance.

The mechanism based on the kinetic model does not take into account the adsorption of $CO_2$ on oxide centers. However, $CO_2$ adsorption may compete with propene transformation into $CO/CO_2$ in which case $\gamma$ will increase. Previous experiments in which $CO_2$ was introduced in the feed demonstrate this effect. It was discovered that it is possible to increase the steady-state relative AA yield (≈10%) by adding an additional percentage of $CO_2$ to the reaction mixture. However, this improvement requires a high excess of $CO_2$ and is limited to a fairly narrow operating temperature domain.

A key feature of the above mechanism is the production of a gaseous intermediate (i.e., propene), which subsequently reacts non-selectively with the catalyst. This process leads to a decrease in catalyst performance. According to one embodiment, the best performance will not reach the relative yield presented in Equation (1) if the system is operated under steady-state conditions.

There are a number of ways to improve the yield of acrylic acid product using the kinetic model:

Under steady-state conditions the catalyst cannot be maintained in a highly oxidized state, and propene production will increase. In this case, the conditions must be adjusted to increase the selectivity of the propene to acrylic process. Based on kinetic studies, the optimal temperature range for steady-state reaction is 375-385°C. The optimum operating conditions are in the regime corresponding to the "upper" branch of hysteresis. To obtain high selectivity an air to propane ratio of 10, addition of up to 20% $CO_2$, and 30 - 40% water is contemplated.

Mechanistic studies indicate that maximum performance is obtained when propane oxidation to acrylic acid occurs at a single site, and the production of gas phase intermediates is minimized. Non steady-state reaction studies indicate that performance is improved by increasing the amount of active oxygen on the catalyst surface.

According to one embodiment, the highest activity and selectivity is achieved by operating under non-steady-state conditions using a two-step process that includes a dual riser reactor with catalyst preoxidation riser and hydrocarbon reaction riser. The initial riser includes oxidation in air or in an air-water mixture at 350 - 370 °C. Water may be added during the oxidation step to enhance the rate of oxidation (the effect of water on catalyst re-oxidation has not been studied in detail, but it appears that the activation energy for oxidation decreases with increasing water concentration). The catalyst inventory is also maintained under oxidizing conditions. The second step includes a riser process in which an air-propane (10/1) mixture, and water are fed to the riser.

Oxygen desorption data indicates that oxygen loss from the surface occurs rapidly, and non steady-state step response data indicates that catalyst performance falls rapidly as the oxidation state is decreased. Thus pre-oxidizing the catalyst immediately before the introduction of propane will provide a greater concentration of active oxygen on the catalyst surface at the time of propane adsorption, and provides a significant boost in yield.

Results from TAP vacuum pulse response experiments indicate that an increase in the Te surface concentration promotes an increase in acrylic acid production and a decrease in acrolein production. Vacuum pulse response experiments also indicate that the rate of water desorption decreases when the surface concentration of tellurium is increased. TAP pulse response experiments and atmospheric pressure step response indicate that water reacts with an adsorbed intermediate to produce acrylic acid. TAP pump probe experiments indicate that there is an optimum time for the introduction of water, which indicates that the adsorbed intermediate can react by another route (e.g., desorption, further oxidation). In the absence of water, propene and acrolein are the principal selective products. Comparison of TAP pulse response experiments using propene with ones using propane indicates that less acrolein is formed from propene than from propane. This result indicates that under TAP conditions acrolein is formed before propene desorption.

Atomic beam deposition of small concentrations of tellurium increased catalyst selectivity, reduced activity, and decreased the rate of water desorption. In our studies, tellurium promoted samples were oxidized at reaction temperature prior to being exposed to a reactant mixture. Tellurium's form on the surface is not known at present, however, it is reasonable to assume that an increase in the Te surface concentration would increase the number of Te centers in the vicinity of the vanadium centers. It is also expected that increased Te concentration could also block some of the vanadium centers. Thus an increase in the Te surface concentration can both increase selectivity and decrease activity.

The rate of water desorption from a MMO1 sample modified by atomic beam deposition (ABD) was compared with a sample modified by wet impregnation. Both samples were enriched with 0.1% Te. The wet impregnated sample was prepared at Rohm and Haas. After deposition, each sample was transferred to a TAP micro-reactor and then pressurized to one atmosphere in air. The sample was then heated to 350 °C in a static pressure of air for 30 minutes, and then an oxygen/argon (8/92 molar ratio) flow for as long as 12 hours. Propene reduction experiments performed after the flow oxidation treatment indicate that complete oxygen uptake on an ABD sample takes one or more hours. TAP pulse response data indicates that water desorbs more slowly from the sample containing 0.1% Te prepared by atomic beam deposition.

To investigate further increases in the Te surface concentration and how treatment conditions influence catalyst performance ≈ 0.1 % Te was deposited on an MMO1 sample containing 0.1% Te prepared by wet impregnation at Rohm and Haas. Performance of the initial wet impregnated MMO1 sample was then compared with the sample containing an additional 0.1% Te. Prior to reaction both samples were heated to 350 °C in a static pressure of air for ≈ 30 minutes. The samples were not exposed to an oxygen flow, and water was not added to the reactant flow. Both samples were tested at steady-state conditions at 350°C using a typical steady-state feed (Pr/$O_2$/Ar 7/14/79 molar ratio) and contact time (1.2 seconds). Before running the steady-state reaction both samples were heated to 350 °C in the reactor in air.

The wet impregnated MMO1 sample showed typical conversion and selectivity when compared with previous results. The sample containing the additional 0.1% Te exhibited lower conversion and similar selectivity. When compared with previous ABD samples containing 0.1% Te this result indicates that the Te surface concentration, which gives the highest performance is in the 0.1 - 0.2% range. Alternatively, the change in the initial oxygen treatment can influence the incorporation of Te into the active site. In the later case it is expected that conversion increases with time on stream. So far, however, this has not been the case.

It is generally agreed that propane activation occurs at a vanadium-oxygen site, but it is not known whether the oxygen species is atomic (1) or molecular (2).

(1)

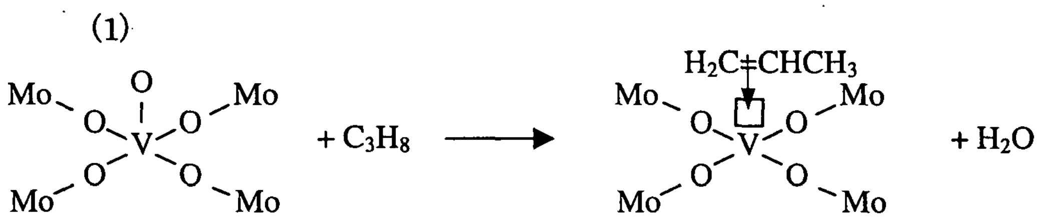


(2)

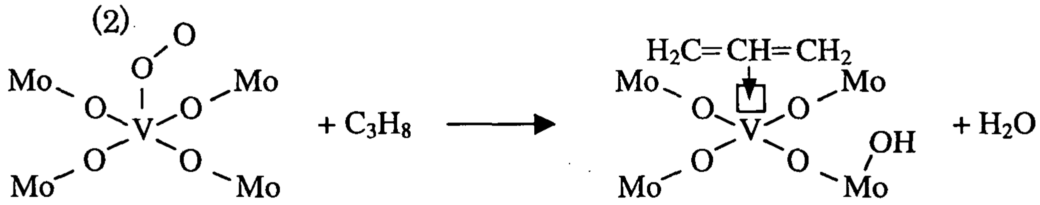


A combination of oxygen isotope and atomic deposition experiments was used to understand how oxygen is activated and to determine the nature of the active oxygen species. A typical set of experiments on a single catalyst sample includes but is not limited to the following three basic experimental sequences.

Sequence 1: A sample of a mixed metal oxide catalyst that has been reaction-equilibrated at steady-state conditions is heated to a fixed temperature under vacuum and exposed to a series of $^{18}O_2$ pulses. The oxygen uptake is determined from the oxygen breakthrough curve. The oxygen-enriched sample is heated (temperature programmed), and the amount of reversibly adsorbed oxygen, and the degree of oxygen exchange is then determined from the TPD spectrum. This sequence is repeated for different fixed oxidation temperatures, and at different oxygen pressures (oxidation at $P_{ox}$ > 1 atm can be performed by operating the microreactor in the high pressure mode).

(1)

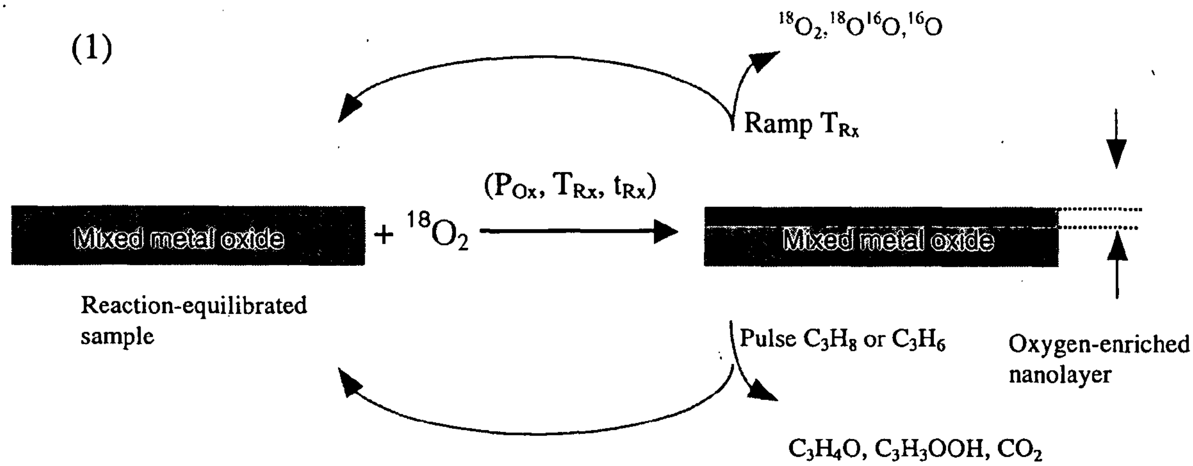


The oxygen-enriched sample is exposed to a series of propane or propene pulses and the primary kinetic characteristics (e.g. apparent rate constants, apparent surface residence time, etc.) the $C_3$ conversion, and the reaction selectivity (to acrolein, acrylic acid, $CO_2$) is determined as a function of pulse number. The reduced sample is reoxidized at the same fixed temperature, and the $C_3$ titration experiment is then repeated at a different temperature. This sequence is repeated for a number of different titration temperatures. The amount of active-selective oxygen, the distribution of oxygen isotopes in the reaction products, and the apparent activation energy will is determined, and equated with the amount of $O_2$ adsorbed by the catalyst.

Sequence 2: Using a calibrated atomic beam, a fixed number of metal atoms are deposited on the surface of a reaction-equilibrated catalyst sample held at room temperature. The modified sample is transferred under vacuum to a TAP microreactor, heated to a fixed temperature and exposed to a series of $^{18}O_2$ pulses. The oxygen uptake along with the amount of reversibly adsorbed oxygen, and the degree of oxygen exchange is then determined. This sequence is repeated for different fixed oxidation temperatures, and at different oxygen pressures.

The oxygen-enriched sample is exposed to a series of propane or propene pulses and the primary kinetic characteristics, $C_3$ conversion, and the reaction selectivity is determined as a function of pulse number. The reduced sample is reoxidized at the same fixed temperature, and the $C_3$ titration experiment is repeated at a different temperature. This sequence is repeated for a number of different titration temperatures. The amount of active-selective oxygen, distribution of oxygen isotopes in reaction products, and the apparent activation energy is then determined, and equated with the amount of $O_2$ adsorbed by the catalyst.

After testing a modified sample, the resulting sample (material (A), or material (B)) is then returned to the atomic beam deposition chamber and another fixed number of metal atoms are deposited on the surface at room temperature. The new modified sample is transferred under vacuum to a TAP microreactor, and the second step of Sequence 2 is repeated. Repeating steps 2 and 3 of Sequence 2 allows one to equate the number of metal atoms deposited with the oxygen uptake, and the amount of active-selective oxygen, and to determine the relationship between the amounts of deposited metal and the selectivity and activity of the catalyst.

(2)

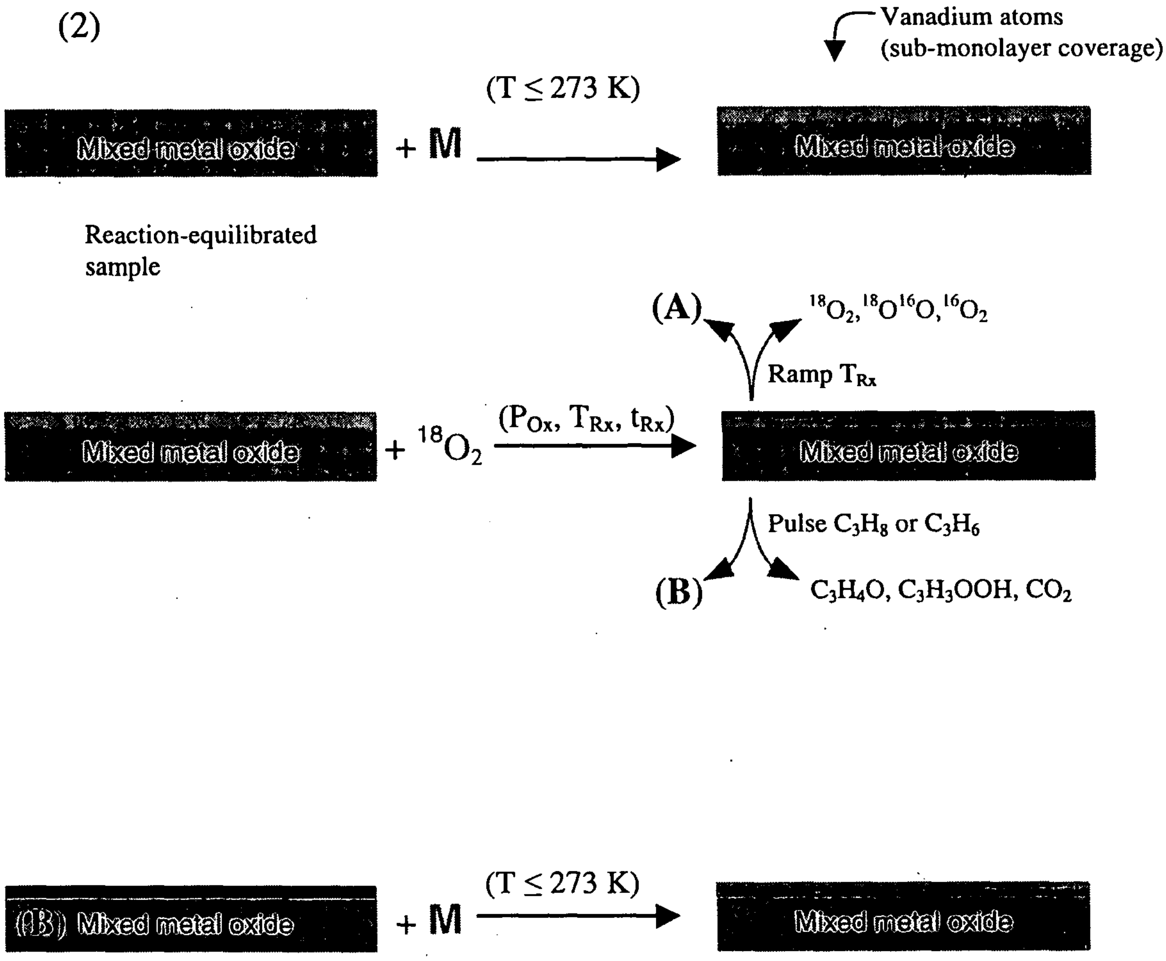


<u>Sequence 3:</u> Using a flow of atomic oxygen a modified sample with a known number of metal atoms, is oxidized at room temperature. After oxidation the sample is then transferred under vacuum to a TAP microreactor, heated to a fixed temperature and exposed to a series of $^{18}O_2$ pulses. The oxygen uptake, amount of reversibly adsorbed oxygen, and the degree of oxygen exchange is determined. The $^{18}O_2$ uptake of samples oxidized with atomic oxygen is then compared with the uptake of freshly modified samples. After exposure to $^{18}O_2$ the primary kinetic characteristics (e.g. apparent rate constants, apparent surface residence time, etc.) $C_3$ conversion, and reaction selectivity is determined as a function of pulse number.

<u>Sequence 3A:</u> According to a separate embodiment, a modified sample is oxidized at room temperature with oxygen atoms, and immediately transferred to a TAP microreactor. The sample is heated (temperature programmed) and the activation energy of propane or propene adsorption is then determined from the series of pulse response curves collected at different temperatures. The sample is reoxidized with atomic oxygen, transferred to a TAP microreactor and heated to a fixed temperature. The primary kinetic characteristics, $C_3$ conversion, etc. is determined as a function of pulse number.

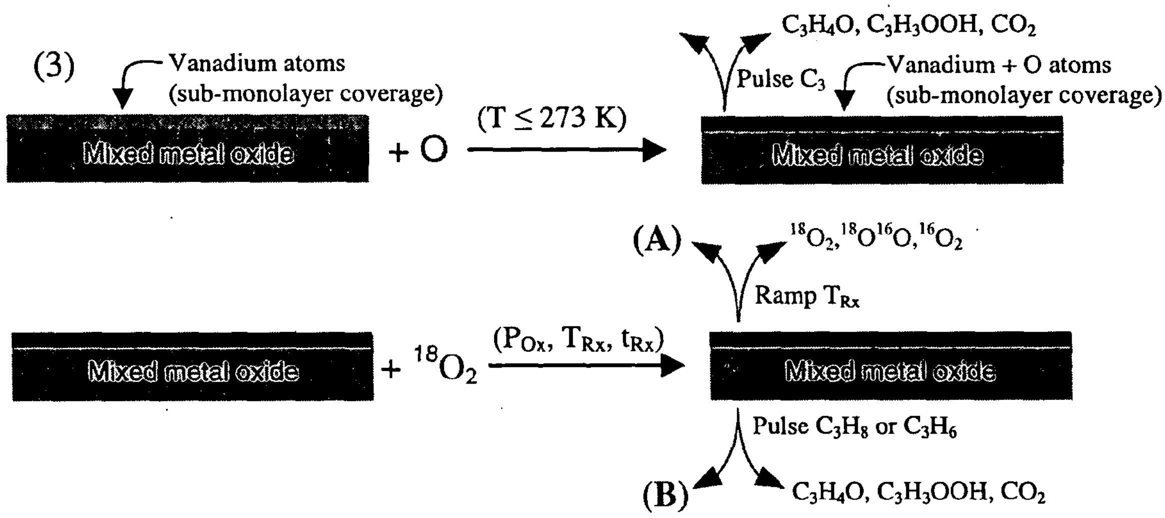


Sequence 3B: According to a separate emobodiment, a reaction-equilibrated sample is exposed to a series of propane or propene pulses, and the kinetic characteristics is determined. The reduced sample is transferred to the deposition chamber and reoxidized using atomic oxygen. The sample is returned to the TAP microreactor, and the primary kinetic characteristics and other parameters is then determined as a function of pulse number.

Experiments performed in Sequences 3, 3A, 3B provide catalytic characteristics of samples activated with atomic oxygen. The results of these experiments are compared with the results from experiments using samples activated with molecular oxygen. The comparison allows one to distinguish the roles of atomically and molecularly adsorbed oxygen species. Determining how the selectivity of different oxide systems changes with metal atom coverage helps distinguish the contribution of the underlying crystal lattice from the contribution of the surface composition. This information is useful for formulating new catalyst systems.

Acrolein production is observed in TAP vacuum pulse response experiments, and in step transient experiments. Preliminary TAP experiments using propene as the reactant indicate that it produces less acrolein then is produced from propane. This result indicates that the formation of acrolein in TAP experiments probably occurs before propene can desorb from the initial propane adsorption site. We assume that acrolein formation initially involves the transfer of a hydrogen atom from the propene intermediate to an adjacent oxygen, and the formation of an allylic intermediate.

Mo-O-V-□ $CH_2$=$C_2H_4$→ Mo-OH-□- $CH_2$= CH $CH_2$

Transport of an oxygen atom via the surface lattice to the adsorbed allylic species, and the transfer of a second hydrogen atom gives acrolein. It is reasonable to assume that the rate of acrolein production will depend on the oxidation state of the catalyst surface, which in turn is a function of the gas phase composition. When the surface is highly oxidized hydrogen transfer is fast, and oxygen transport to the adsorbed allylic intermediate is fast. In this case acrolein can be formed before the propene intermediate can desorbs. In TAP vacuum pulse response experiments, acrolein production is observed after the catalyst has been oxidized.

Mo-OH-V-□ $CH_2$= CH $CH_2$→ [Mo-OH, Mo-□ ]-V-$OC_3H_5$

[Mo-OH, Mo-□ ]-V-$OC_3H_5$→ [Mo-OH, Mo-OH, Mo-□ ] V-□ + $C_3H_4O$

After desorption of acrolein the active site is regenerated by reaction with gaseous oxygen.

Mo-□ -V-□ + $O_2$→ Mo-O-VO

Under conditions in which the surface oxidation state is low, propene desorption can occur before acrolein is formed. Under steady-state conditions, when the catalyst is exposed to a mixture of oxygen and propane the surface oxidation state is lower than in TAP experiments. In this case propene desorption can occur before acrolein is formed. Under steady-state conditions propene production is observed at short contact times, but only trace amounts of acrolein are observed.

At present it is not fully known how acrolein reacts with an oxidized surface, and this is explored to determine

the optimum conditions for acrolein conversion under nonsteady-state conditions.

If propene desorbs before it is converted to an allylic intermediate it can readsorb at another defect site or react with an active oxygen species. At the same steady-state conditions propene conversion to acrylic acid is ≈ 1/2 as selective as acrolein conversion. Consequently increased selectivity to acrylic acid can be achieved if propene can be converted to acrolein before it desorbs.

TAP pump-probe experiments show that in the presence of water acrolein is rapidly converted to acrylic acid. Water also enhances the rate acrolein production in TAP experiments. Water adsorption occurs at oxygen surface vacancies, which may be relatively high in number at reaction conditions. Upon adsorption water can transfer a hydrogen atom to an adjacent oxygen species, and modify its bond with adjoining metal cations. Adsorbed water increases the fluidity of surface species, which can explain why its increased concentration increases the rate of product formation. The water adsorption sites are also potential sites for propene readsorption. Thus water adsorption may compete with propene re-adsorption, and decrease the adsorption of propene at nonselective sites.

At present it is not known how acrylic acid reacts with an oxidized surface, and this is explored to determine the optimum conditions for acrolein conversion under nonsteady-state conditions.

Chemical treatments, resulting in treated/modified catalysts, include one or more chemical modifying agents. Suitable chemical modifying agents include, but are not limited to for example, oxidizing agents selected from hydrogen peroxide, nitrogen, nitric acid, nitric oxide, nitrogen dioxide, nitrogen trioxide, persulfate; reducing agents selected from amines, pyridine, hydrazine, quinoline, metal hydrides, sodium borohydride, C1-C4 alcohols, methanol, ethanol, sulfites, thiosulfites, aminothiols; combinations of oxidizing agents and reducing agents; acids selected from HCl, HN03, H2SO4; organic acids, organic diacids, acetic acid, oxalic acid, combinations of C1-C4 alcohols and C1-C4 organic acids, oxalic acid and methanol; inorganic bases selected from NH3, NH40H, H2NNH2, HONH2, NaOH, Ca(OH)2, CaO, Na2CO3, NaHC03, organic bases selected from ethanol amine, diethanolamine, triethanolamine; pH adjustments; peroxides selected from inorganic peroxides, H2O2, organic peroxides, tBu2O2; chelating agents, ethylenediamine, ethylenediaminetetraacetic acid (EDTA); electrolysis including electrolytic reduction; treatment with high energy radiation including ultraviolet and X-ray radiation; and combinations thereof.

Physical treatments, resulting in treated/modified catalysts, include one or more physical processes. Suitable physical processes include, but are not limited to for example, cooling, cryogenic cooling, pressure cooling, compacting under pressure, high pressure die pressing, thermolyzing (also referred to as polymer burn off), mechanical grinding at cryogenic temperatures, high shear grinding at cryogenic temperatures, cryo-milling, cryo-densifying, cryo-stressing, cryo-fracturing, cryo-pelletizing, deforming, wash coating, molding, forming, shaping, casting, machining, laminating, drawing, extruding, lobalizing, impregnating, forming spheres (spherolizing or jetting), slurrying, cryo-slurrying, preparing shelled catalysts (shelling), multi-coating, electrolyzing, electrodepositing, compositing, foaming, cryo-fluidizing, cryo-spraying, thermal spraying, plasma spraying, vapor depositing, adsorbing, ablating, vitrifying, sintering, cryo-sintering, fusing, fuming, crystallizing, any altering of catalyst crystal structure, polycrystallizing, recrystallizing, any surface treating of the catalyst, any altering of catalyst surface structure, any altering of catalysts porosity, any altering of catalyst surface area, any altering of catalyst density, any altering of bulk catalysts structure, reducing the particle size of the primary catalyst particles in combination with cooling or thermolyzing the catalyst, and any combinations of chemical and physical treatments, including but not limited to solvent extraction, Soxhlet extraction, batch solvent extraction, continuous flow solvent extraction, extraction in supercritical solvents, contacting the catalyst with one or more leaching agents including solvents, altering catalyst pH, any chemical treatments used in modifying catalyst surface structure, mechanical grinding in supercritical solvents, chemisorbing one or more chemical agents, ultrasonification using one or more solvents selected from organic solvents such as alcohols and amines ultrasonification, and any physical treatments employing solvents under supercritical conditions. According to a separate embodiment, modified catalysts include one or more further chemical and/or physical treatments of already modified catalysts.

According to one embodiment, modified catalysts are further modified by one or more physical treatments including, but not limited to for example, heating, drying, cooling, freeze, pressure cooling, thermal die pressing, high pressure die pressing, thermal and high pressure die pressing, thermal high shear milling and grinding, thermal depolymerizing, thermolyzing (also referred to as polymer burn off), mechanical grinding at cryogenic temperatures, mechanical grinding at elevated temperatures, thermal milling, cryo-milling, thermal shearing, cryo-shearing, cryo-densifying, densification, coagulation, flocculation, sedimenting, lyophilizing, agglomerating, reducing particle size of primary particles, increasing surface area of primary particles, thermal and cryo-compacting, thermal and cryo-compressing, thermal and cryo-stressing, cryo-fracturing, shear loading, thermal and cryo-shear loading, drawing, thermal and cryo-drawing, thermal and cryo-centrifuging, thermal and cryo-granulating, thermal and cryo-spray drying, atomizing, thermal and cryo-dry pressing, cryo-pressing, heat pressing, dry compacting, cryo-compacting, heat compacting, isocompacting, thermal and cryo-isocompacting, thermal and cryo-pelletizing, thermal and cryo-roll pressing, thermal and cryo-deforming, jiggering, thermal and cryo-molding, thermal and cryo-forming, thermal and cryo-shaping, thermal and cryo-casting, thermal and cryo-machining, thermal and cryo-laminating, thermal and cryo-tape casting, fiber drawing, thermal and cryo-fiber drawing, thermal and cryo-fiber extruding, thermal and cryo-extruding, thermal and cryo-lobalizing, thermal

and cryo-impregnating, forming sphere forming (spherolizing or jetting), slurrying, cryo-slurrying, preparing shelled catalysts (shelling), multi-coating, electrolyzing, electrodepositing, compositing, rolling, roll forming, foaming, cementing, fluidizing, cryo-spraying, thermal spraying, plasma spraying, vapor depositing, adsorbing, ablating, firing, vitrifying, sintering, cryo-sintering, pre-shaping before extruding, thermal and cryo-pre-shaping before extruding, lobalizing, fusing, thermal fusing, fuming, coking, colloidalizing, crystallizing, thermal and cryo-crystallizing, any altering of crystal structure, polycrystallizing, recrystallizing, any surface treating, any altering of surface structure, any altering of porosity, any altering of density, any altering of bulk structure, altering catalyst pH, any chemical treatments used in modifying catalyst surface structure, mechanical grinding in supercritical solvents, chemisorbing one or more chemical agents, ultrasonification using one or more solvents selected from organic solvents, aqueous solvents and combinations of organic and aqueous solvents including, but limited to for example, acids, alcohols, chelating agents and amines ultrasonification, and any physical treatments employing solvents under supercritical conditions and any combinations thereof.

Other suitable treatments involve combinations of one or more chemical modifying agents and one or more physical processes, resulting in treated/modified catalysts. Suitable examples include, but are not limited to for example, solvent extraction using a Soxhlet extractor, extraction using a Parr bomb, solvent extraction using microwave radiation, batch solvent extraction, continuous flow solvent extraction, leaching, altering pH, any surface treatments, grinding in supercritical solvents, extraction in supercritical solvents, chemisorption, ultrasonification using one or more solvents selected from organic solvents such as alcohols and amines; and combinations thereof.

According to one embodiment of the invention, modified mixed metal oxides useful as catalysts in alkane oxidations are prepared by mechanical grinding unmodified (prepared) mixed metal oxide catalysts at cryogenic temperatures. Cryogenic temperatures are meant to refer to temperatures between 10°C (283 K) to -269°C (4 K). Catalysts are cryo-ground using a suitable cryogen source in combination with suitable corresponding milling equipment. Suitable examples include, but are not limited to for example, freeze milling using a freezer mill, and any milling at cryogenic temperatures. Such cryo-grinding affords modified mixed metal oxide catalysts and the resulting performance characteristics of the modified catalysts are improved selectivities and yields at constant alkane, alkene or alkane and alkene conversion. For example, cryo-milling mixed metal oxide catalysts having the empirical formula

$$MoV_aNb_bX_cZ_dO_n$$

wherein X is at least one element selected from the group consisting of Te and Sb, Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements, $0.1 \leq a \leq 1.0$, $0.01 \leq b \leq 1.0$, $0.01 \leq c \leq 1.0$, $0 \leq d \leq 1.0$ and n is determined by the oxidation states of the other elements, provides modified mixed metal oxide catalysts whose catalytic performance results in significantly improved acrylic acid (AA) selectivities and yield at constant propane conversion as compared to corresponding unmodified mixed metal oxide catalysts or as compared to simply milling the corresponding unmodified mixed metal oxide catalysts using conventional mechanical grinding equipment.

According to a separate embodiment of the invention, modified mixed metal oxides useful as catalysts in alkane oxidations are prepared by treating corresponding unmodified (prepared) mixed metal oxide catalysts with one or more chemical modifying agents, namely one or more reducing agents. Suitable reducing agents include, for example, reducing agents selected from primary amines, secondary amine, tertiary amines, alkylamines, dialkylamines, trialkyl-and triaryl amines, methylamine, dimethylamine, trimethylamine, pyridine, hydrazine, quinoline, metal hydrides, sodium borohydride, C1-C4 alcohols, methanol, ethanol, sulfites, thiosulfites, aminothiols, combinations of oxidizing agents and reducing agents, NH3, NH40H, H2NNH2, HONH2, ethanol amine, diethanolamine, triethanolamine, adjusting to pH > 7, electrolysis including electrolytic reduction and combinations thereof. Such post treatment affords modified mixed metal oxide catalysts and the resulting performance characteristics of the modified catalysts are improved selectivities and yields at constant alkane, alkene or alkane and alkene conversion. For example, modified mixed metal oxide catalysts having the empirical formula:

$$M_eMoV_aNb_bX_cZ_dO_n$$

wherein $M_e$ is at least one or more chemical modifying agents, X is at least one element selected from the group consisting of Te and Sb, Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements, $0.1 \leq a \leq 1.0$, $0.01 \leq b \leq 1.0$, $0.01 \leq c \leq 1.0$, $0 \leq d \leq 1.0$ and n, e are determined by the oxidation states of the other elements, using pyridine as a reducing agent results in significantly improved acrylic acid (AA) selectivities and yield at constant propane conversion as compared to corresponding unmodified mixed metal oxide catalysts.

According to a separate embodiment of the invention, modified mixed metal oxides useful as catalysts in alkane oxidations are prepared by a combination of cryo-grinding unmodified mixed metal oxide catalysts followed by solvent extraction of corresponding modified mixed metal oxide catalysts. Catalysts are cryo-ground using a suitable cryogen

source in combination with suitable corresponding milling equipment. Suitable examples include, but are not limited to for example, freeze milling using a freezer mill, and any milling at cryogenic temperatures. Extraction of the modified metal catalysts is subsequently performed using conventional extraction equipment, including for example Soxhlet extractors or Parr bomb extractors using suitable organic solvents, aqueous solvents and combinations of organic and aqueous solvents. Suitable organic solvents include for example C1-C4 alcohols, combinations of C1-C4 alcohols and C1-C6 organic acids/diacids and combinations of C1-C4 alcohols and C1-C6 organic bases. Suitable aqueous solvents include, but are not limited to for example, acids, bases ,chelating agents and combinations thereof. The combination of cryo-grinding followed by solvent extraction affords modified mixed metal oxide catalysts and the resulting performance characteristics of the modified catalysts are improved selectivities and yields at constant alkane, alkene or alkane and alkene conversion. For example, cryo-milling mixed metal oxide catalysts having the empirical formula

$$MoV_aNb_bX_cZdO_n$$

wherein X is at least one element selected from the group consisting of Te and Sb, Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements, $0.1 \leq a \leq 1.0$, $0.01 \leq b \leq 1.0$, $0.01 \leq c \leq 1.0$, $0 \leq d \leq 1.0$ and n is determined by the oxidation states of the other elements, followed by solvent extraction of the corresponding modified catalysts results in significantly improved acrylic acid (AA) selectivities and yield at constant propane conversion as compared to corresponding unmodified mixed metal oxide catalysts or as compared to simply grinding the corresponding unmodified mixed metal oxide catalysts using conventional mechanical grinders.

Solvent extraction is carried out in a batch process or using continuous solvent flow extraction. Modified catalyst particles are slurried in to an extraction medium comprising one or more organic solvents, typical alcohols. Other organic solvents are also usefully employed. The extraction process is carried out for deliberate periods of time in conventional equipment including for example a Soxhlet extractor, a Parr bomb reactor heated to a suitable temperature and pressure, heated by convection or using microwave radiation. One characteristic of both types of solvent extractions is that the catalyst particles are in constant contact with the extraction solvent. As the extraction process proceeds with time, the concentration of dissolved materials extracted into solvent increases until a chemical equilibrium is reached. One advantage of continuous solvent flow extraction is that the catalyst particles are not in contact with the bulk of the solvent. Dissolved or extracted materials accumulate in the bulk solvent vessel and evaporation and condensation of the solvent insures a solvent containing no dissolved material for extraction. The continuous solvent flow extraction method is carried out in open systems at atmospheric pressure or closed systems under pressure. Furthermore, there is no need for washing the catalyst particles with additional new solvents after extraction nor is there the need for filtration in order to separate the catalysts particles from the extraction solvent. Suitable extraction solvents include but are not limited to single phase solvents. Suitable solvents include for example water, C1-C4 alcohols, C1-C6 organic acids and diacids, C1-C6 amines, chelating agents and combinations thereof.

According to a separate embodiment of the invention, modified mixed metal oxides useful as catalysts in alkane oxidations are prepared by a combination of ultrasonification of unmodified mixed metal oxide catalysts in one or more organic solvents, aqueous solvents and combinations of organic and aqueous solvents. In a related separate embodiment, ultrasonifaction is combined with solvent extraction of corresponding modified mixed metal oxide catalysts. Catalysts are milled ultrasonically using conventional ultrasonfication equipment. The ultrasonicator is equipped with a cryogen source and a heating source. Extraction of the modified metal catalysts is subsequently performed using conventional extraction equipment, including for example Soxhlet extractors using suitable organic solvents. Suitable organic solvents include for example C1-C4 alcohols, combinations of C1-C4 alcohols and C1-C6 organic acids/diacids, combinations of C1-C4 alcohols and one or more chelating agents, combinations of C1-C4 alcohols and C1-C6 organic bases and corresponding combinations thereof. Ultrasonification in one or more solvents and the combination of ultrasonification followed by solvent extraction affords modified mixed metal oxide catalysts whose catalytic performance characteristics results in improved selectivities and yield at constant propane conversion as compared to corresponding unmodified mixed metal oxide catalysts or as compared to simply grinding the corresponding unmodified mixed metal oxide catalysts using conventional mechanical grinders.

According to a separate embodiment of the invention, modified mixed metal oxides useful as catalysts in alkane oxidations are prepared by densifying the catalysts by pressure compacting or cryo-milling. Catalysts are pressure compacted using conventional compaction equipment. The pressure compactor is optionally equipped with a cryogen source and a heating source. Compaction of catalysts under compacting loads affords modified mixed metal oxide catalysts and the resulting performance characteristics of the modified catalysts are improved selectivities and yield at constant propane conversion as compared to corresponding unmodified mixed metal oxide catalysts. Modified MMO catalysts exhibit higher AA yields as compared to unmodified MMO catalysts. For example, a 0.2 to 0.3 $g/cm^3$ increase in catalyst density increases AA yield up 5 %. Cryo-grinding was found to provide an 0.15 to 0.20 $g/cm^3$ increase in packed density of selected modified MMO catalysts. In another example, AA yields from higher density cryo-milled

modified MMO catalysts were 2-4% (absolute) higher. Surface area data of selected modified MMO catalysts have higher surface areas (13 $m^2/g$) as compared to unmodified and conventionally milled MMO catalysts (6 to 11 $m^2/g$), accounting for the AA yield increase.

According to a separate embodiment of the invention, modified mixed metal oxides useful as catalysts in alkane oxidations are prepared by a combination of solvent extraction of unmodified mixed metal oxide catalysts in one or more supercritical solvents. In a related separate embodiment, a modified catalyst as compared with conventional preparation is prepared under supercritical conditions. Conventional equipment is used to create supercritical solvent conditions. Suitable examples of supercritical solvents include, but are no limited to for example, C02, H20, NH3, CH30H and ethanol. The supercritical solvent modified catalysts are optionally solvent extracted or further processed using conventional techniques described herein. Supercritical solvent extraction of the modified metal catalysts is subsequently performed using conventional supercritical extraction equipment using suitable organic solvents. Suitable organic solvents include for example, water, carbon dioxide, ammonia, C1-C4 alcohols, combinations of C1-C4 alcohols and C1-C6 organic acids/diacids and combinations of C1-C4 alcohols and C1-C6 organic bases. Supercritical modification of MMO catalysts in one or more solvents and the combination of supercritical solvent extraction followed by affords further modification including, but not limited to heating and milling of the modified mixed metal oxide catalysts and the resulting catalytic performance characteristics of the modified catalysts are improved selectivities and yield at constant propane conversion as compared to corresponding unmodified mixed metal oxide catalysts or as compared to simply grinding the corresponding unmodified mixed metal oxide catalysts using conventional mechanical grinding equipment.

According to a separate embodiment, unmodified MMO catalysts are treated with a source of $NO_x$. In a preferred embodiment, the treatment is performed by further admixing the precursor admixture with a fluid for introducing $NO_x$ to the precursor admixture and then drying or calcining the resulting admixture. Accordingly, preferably the fluid includes a $NO_x$ source such as nitric acid, ammonium nitrate, ammonium nitrite, NO, $NO_2$ or a mixture thereof. More preferably, the fluid is a liquid, such as an aqueous solution, including the $NO_x$ source dissolved or dispersed therein. In another embodiment, it is contemplated that a gas including a source of $NO_x$ is bubbled or otherwise introduced into the precursor admixture for treating the admixture. For example, the precursor admixture prior to calcination is prepared by mixing the precursor admixture and nitric acid solution to form a resulting admixture having 0.01 to 20 percent by weight of nitric acid, and more preferably 0.05 to 10 percent by weight of nitric acid. In another example, the resulting admixture has 0.1 to 1.5 percent by weight of nitric acid. Alternatively expressed, prior to calcination, preferably the nitric acid is present in an amount of at least 500 ppm of the admixture, more preferably, at least 1500 ppm. An example of a preferred range of concentrations includes 1000 to 15,000 ppm nitric acid.

In another embodiment, where the source of $NO_x$ includes $NO_2$, the amount of $NO_2$ ranges from 500 to 12,000 ppm and more preferably 1000 to 9000 ppm.

Once the resulting modified or treated catalysts are formed, liquid therein is removed by any suitable method, known in the art, for forming a catalyst precursor. Such methods include, without limitation, vacuum drying, freeze drying, spray drying, rotary evaporation and air-drying. Vacuum drying is generally performed at pressures ranging from 10 mm Hg to 500 mm Hg. Freeze drying typically entails freezing the slurry or solution, using , for instance, liquid nitrogen, and drying the frozen slurry or solution under vacuum. Spray drying is generally performed under an inert atmosphere such as nitrogen or argon, with an inlet temperature ranging from 125°C to 200°C and an outlet temperature ranging from 75°C to 150°C. Rotary evaporation is generally performed at a bath temperature of from 25°C to 90°C and at a pressure of from 10 mm Hg to 760 mm Hg, preferably at a bath temperature of from 40° to 90°C and at a pressure of from 10 mm Hg to 350 mm Hg, more preferably at a bath temperature of from 40°C to 60°C and at a pressure of from 10 mm Hg to 40 mm Hg. Air drying may be effected at temperatures ranging from 25°C to 90°C. Rotary evaporation or air-drying are generally preferred.

Once obtained, the resulting modified catalyst precursor is used as modified or is further modified by conventional processes well known in the art, including further milling and calcining.

According to one embodiment, calcination may be conducted in an oxygen-containing atmosphere or in the substantial absence of oxygen, e.g., in an inert atmosphere or in vacuo. The inert atmosphere may be any material which is substantially inert, i.e., does not react or interact with, the catalyst precursor. Suitable examples include, without limitation, nitrogen, argon, xenon, helium or mixtures thereof. Preferably, the inert atmosphere is argon or nitrogen. The inert atmosphere may flow over the surface of the catalyst or may not flow thereover (a static environment). When the inert atmosphere does flow over the surface of the catalyst precursor, the flow rate can vary over a wide range, e.g., at a space velocity of from 1 to 500 $hr^{-1}$.

Calcination of both unmodified and modified catalysts is usually performed at a temperature of from 350°C to 850°C, preferably from 400°C to 700°C, more preferably from 500°C to 640°C. The calcination is performed for an amount of time suitable to form the aforementioned catalyst. Typically, the calcination is performed for from 0.5 to 30 hours, preferably from 1 to 25 hours, more preferably for from 1 to 15 hours, to obtain the desired promoted mixed metal oxide. According to one embodiment, the unmodified and modified catalyst is calcined in two stages. In the first stage, the catalyst precursor is calcined in an oxidizing environment (e.g. air) at a temperature of from 200°C to 400°C, preferably

from 275°C to 325°C for from 15 minutes to 8 hours, preferably for from 1 to 3 hours. In the second stage, the material from the first stage is calcined in a non-oxidizing environment (e.g., an inert atmosphere) at a temperature of from 500°C to 700°C, preferably for from 550°C to 650°C, for 15 minutes to 8 hours, preferably for from 1 to 3 hours. Optionally, a reducing gas, such as, for example, ammonia or hydrogen, may be added during the second stage calcination.

According to a separate embodiment, a modified metal oxide catalyst is obtained through cryo-grinding (also referred to a freeze milling). There is no particular restriction as to the grinding method, and conventional methods may be employed. As a dry grinding method, a method of using a gas stream grinder may, for example, be mentioned wherein coarse particles are permitted to collide with one another in a high speed gas stream for grinding. The grinding may be conducted not only mechanically but also by using a mortar or the like in the case of a small scale operation.

As a wet grinding method wherein grinding is conducted in a wet state by adding water or an organic solvent to the above mixed metal oxide, a conventional method of using a rotary cylinder-type medium mill or a medium-stirring type mill, may be mentioned. The rotary cylinder-type medium mill is a wet mill of the type wherein a container for the object to be ground is rotated, and it includes, for example, a ball mill and a rod mill. The medium-stirring type mill is a wet mill of the type wherein the object to be ground, contained in a container is stirred by a stirring apparatus, and it includes, for example, a rotary screw type mill, and a rotary disc type mill.

The conditions for grinding may suitably be set to meet the nature of the above-mentioned promoted mixed metal oxide, the viscosity, the concentration, etc. of the solvent used in the case of wet grinding, or the optimum conditions of the grinding apparatus. However, it is preferred that grinding is conducted until the average particle size of the ground catalyst precursor would usually be at most 20$\mu$m, more preferably at most 5$\mu$m. Improvement in the catalytic performance occurs due to such cryo-grinding.

Further, in some cases, it is possible to further improve catalytic activities by further adding a solvent to the ground catalyst precursor to form a solution or slurry, followed by drying again. There is no particular restriction as to the concentration of the solution or slurry, and it is usual to adjust the solution or slurry so that the total amount of the starting material compounds for the ground catalyst precursor is from 10 to 60 wt. %. Then, this solution or slurry is dried by a method such as spray drying, freeze drying, evaporation to dryness or vacuum drying, preferably by the spray drying method. Further, similar drying may be conducted also in the case where wet grinding is conducted.

The modified mixed metal oxide (promoted or not) obtained by the above-mentioned method may be used as a final catalyst, but it may further be subjected to one or more additional chemical, physical and combinations of chemical and physical treatments. According to one embodiment, modified catalysts are further modified using heat treatment. As an exemplary embodiment, heat treatment usually is performed at a temperature of from 200° to 700°C for from 0.1 to 10 hours.

The resulting modified mixed metal oxide (promoted or not) may be used by itself as a solid catalyst. The modified catalysts are also combined with one or more suitable carriers, such as, without limitation, silica, alumina, titania, aluminosilicate, diatomaceous earth or zirconia, according to art-disclosed techniques. Further, it may be processed to a suitable shape or particle size using art disclosed techniques, depending upon the scale or system of the reactor. Alternatively, the metal components of the modified catalysts are supported on materials such as alumina, silica, silica-alumina, zirconia, titania, etc. by conventional incipient wetness techniques. In one typical method, solutions containing the metals are contacted with the dry support such that the support is wetted; then, the resultant wetted material is dried, for example, at a temperature from room temperature to 200°C followed by calcination as described above. In another method, metal solutions are contacted with the support, typically in volume ratios of greater than 3 : 1 (metal solution : support), and the solution agitated such that the metal ions are ion-exchanged onto the support. The metal-containing support is then dried and calcined as detailed above.

According to a separate embodiment, modified catalysts are also prepared using one or more promoters. The starting materials for the above promoted mixed metal oxide are not limited to those described above. A wide range of materials including, for example, oxides, nitrates, halides or oxyhalides, alkoxides, acetylacetonates, and organometallic compounds may be used. For example, ammonium heptamolybdate may be utilized for the source of molybdenum in the catalyst. However, compounds such as $MoO_3$, $MoO_2$, $MoCl_5$, $MoOCl_4$, $Mo(OC_2H_5)_5$, molybdenum acetylacetonate, phosphomolybdic acid and silicomolybdic acid may also be utilized instead of ammonium heptamolybdate. Similarly, ammonium metavanadate may be utilized for the source of vanadium in the catalyst. However, compounds such as $V_2O_5$, $V_2O_3$, $VOCl_3$, $VCl_4$, $VO(OC_2H_5)_3$, vanadium acetylacetonate and vanadyl acetylacetonate may also be utilized instead of ammonium metavanadate. The tellurium source may include telluric acid, $TeCl_4$, $Te(OC_2H_5)_5$, $Te(OCH(CH_3)_2)_4$ and $TeO_2$. The niobium source may include ammonium niobium oxalate, $Nb_2O_5$, $NbCl_5$, niobic acid or $Nb(OC_2H_5)_5$ as well as the more conventional niobium oxalate.

In addition, with reference to the promoter elements for the promoted catalyst, the nickel source may include nickel(II) acetate tetrahydrate, $Ni(NO_3)_2$, nickel(II) oxalate, NiO, $Ni(OH)_2$, $NiCl_2$, $NiBr_2$, nickel(II) acetylacetonate, nickel (II) sulfate, NiS or nickel metal. The palladium source may include $Pd(NO_3)_2$, palladium(II) acetate, palladium oxalate, PdO, $Pd(OH)_2$, $PdC1_2$, palladium acetylacetonate or palladium metal. The copper source may be copper acetate, copper acetate monohydrate, copper acetate hydrate, copper acetylacetonate, copper bromide, copper carbonate, copper

chloride, copper chloride dihydrate, copper fluoride, copper formate hydrate, copper gluconate, copper hydroxide, copper iodide, copper methoxide, copper nitrate, copper nitrate hydrate, copper oxide, copper tartrate hydrate or a solution of copper in an aqueous inorganic acid, e.g., nitric acid. The silver source may be silver acetate, silver acetylacetonate, silver benzoate, silver bromide, silver carbonate, silver chloride, silver citrate hydrate, silver fluoride, silver iodide, silver lactate, silver nitrate, silver nitrite, silver oxide, silver phosphate or a solution of silver in an aqueous inorganic acid, e.g., nitric acid. The gold source may be ammonium tetrachloroaurate, gold bromide, gold chloride, gold cyanide, gold hydroxide, gold iodide, gold oxide, gold trichloride acid and gold sulfide.

Modified catalysts of the invention have different chemical, physical and performance characteristics in catalytic reactions of carbon based molecules as compared to unmodified catalysts. According to one embodiment, the treated catalyst exhibits changes in X-ray lines, peak positions and intensity of such lines and peaks as compared with corresponding X-ray diffraction data for corresponding unmodified catalysts. Such difference indicate structural differences between the modified and unmodified catalysts and are born out in the catalytic activity and selectivity. For example, compared with an untreated catalyst composition, a treated catalyst composition of the present invention exhibits an X-ray diffraction pattern having a relative increase in a diffraction peak at a diffraction angle (2θ) of 27.1 degrees when compared with an untreated catalyst, which may exhibit no peak at all at 27.1 degrees.

The relative difference between peak intensities of treated versus untreated compositions may be greater than 5%, more preferably greater than 10%, and still more preferably greater than 20% of the intensity of the untreated catalyst composition at the diffraction angle (2θ) of 27.1 degrees. Without intending to be bound by theory, it is believed that at least two phases (A and B) are present in the resulting mixed metal oxide catalyst and the treatment of the catalyst precursor with a source of $NO_x$ results in an increase in phase B relative to phase A in the resulting catalyst. The increase in phase B is believed to contribute to improved performance of the catalyst in terms of selectivity, reactivity and yield.

Modified catalysts of the invention exhibit improved catalyst performance characteristics selected from the group consisting of: optimized catalyst properties, yields of oxygenates including unsaturated carboxylic acids, from their corresponding alkanes, alkenes or combinations of corresponding alkanes and alkenes at constant alkane/alkene conversion, selectivity of oxygenate products, including unsaturated carboxylic acids, from their corresponding alkanes, alkenes or combinations of corresponding alkanes and alkenes, optimized feed conversion, cumulative yield of the desired oxidation product, optimized reactant/product recycle conversion, optimized product conversion via recycle and combinations thereof, as compared to the unmodified catalyst.

Modified catalysts of the invention have improved performance characteristics as compared to unmodified catalysts in catalytic processes comprising any carbon containing molecule. According to one embodiment of the invention, the modified catalysts have improved performance characteristics as compared to unmodified catalysts in processes for preparing dehydrogenated products and oxygenated products from alkanes and oxygen, alkenes and oxygen and combination of alkanes, alkenes and oxygen. The reactions are typically carried out in conventional reactors with the alkanes catalytically converted at conventional residence times (> 100 milliseconds) in conventional reactors. According to a separate embodiment the reactions are carried out at short contact times (≤ 100 milliseconds) in a short contact time reactor. Suitable alkanes include alkanes having straight or branched chains. Examples of suitable alkanes are $C_2$-$C_{25}$ alkanes, including $C_2$-$C_8$ alkanes such as propane, butane, isobutane, pentane, isopentane, hexane and heptane. Particularly preferred alkanes are propane and isobutane.

Modified catalysts of the invention convert alkanes, alkenes or alkanes and alkenes to their corresponding alkenes and oxygenates including saturated carboxylic acids, unsaturated carboxylic acids, esters thereof, and higher analogue unsaturated carboxylic acids and esters thereof. The modified catalyst and catalytic systems are designed to provide specific alkenes, oxygenates and combinations thereof. Alkanes are catalytically converted to one or more products in a single pass, including corresponding alkenes. Any unreacted alkane, alkene or intermediate is recycled to catalytically convert it to its corresponding oxygenate. According to one embodiment, alkenes produced from dehydrogenation of corresponding alkanes using catalyst systems of the invention are deliberately produced as in-process chemical intermediates and not isolated before selective partial oxidation to oxygenated products. For example, when catalytically converting an alkane to its corresponding ethylenically unsaturated carboxylic acid, any unreacted alkene produced is recovered or recycled to catalytically convert it to its corresponding ethylenically unsaturated carboxylic acid product stream.

According to a separate embodiment, alkanes, alkenes or alkanes and alkenes are also catalytically converted to its corresponding oxygenates through two or more catalytic zones. For example, an alkane is catalytically converted to its corresponding saturated carboxylic acid in a first catalytic zone or layer of a mixed catalyst bed. The saturated carboxylic acid, in the presence of an additional formaldehyde stream, to its corresponding higher analogue ethylenically unsaturated carboxylic acid in a second catalytic zone or layer of a mixed bed catalyst. In a specific example, propane is catalytically converted to propionic acid and the propionic acid in the presence of formaldehyde is catalytically converted to methacrylic acid.

As used herein, the term "higher analogue unsaturated carboxylic acid" and "ester of a higher analogue unsaturated carboxylic acid" refer to products having at least one additional carbon atom in the final product as compared

to the alkane or alkene reactants. For example given above, propane ($C_3$ alkane) is converted to propionic acid ($C_3$ saturated carboxylic acid), which in the presence of formaldehyde is converted to its corresponding higher analogue ($C_4$) carboxylic acid, methacrylic acid using catalysts of the invention.

Suitable alkenes used in the invention include alkenes having straight or branched chains. Examples of suitable alkenes include $C_2$-$C_{25}$ alkenes, preferably $C_2$-$C_8$ alkenes such as propene (propylene), 1-butene (butylene), 2-methylpropene (isobutylene), 1-pentene and 1-hexene. Particularly preferred alkenes are propylene and isobutylene.

Suitable aldehydes used in the invention include for example formaldehyde, ethanal, propanal and butanal.

Modified catalysts and catalyst systems of the invention convert alkanes, alkenes or alkanes and alkenes to their corresponding oxygenates including saturated carboxylic acids having straight or branched chains. Examples include $C_2$-$C_8$ saturated carboxylic acids such as propionic acid, butanoic acid, isobutyric acid, pentanoic acid and hexanoic acid. According to one embodiment, saturated carboxylic acids produced from corresponding alkanes using catalyst systems of the invention are deliberately produced as in-process chemical intermediates and not isolated before selective partial oxidation to oxygenated products including unsaturated carboxylic acids, esters of unsaturated carboxylic acids, and higher esters of unsaturated carboxylic acids. According to a separate embodiment, any saturated carboxylic acid produced is converted using catalysts of the invention to its corresponding product stream including an ethylenically unsaturated carboxylic acid, esters thereof, a higher analogue unsaturated carboxylic acid or esters thereof.

Modified catalysts and catalyst systems of the invention also convert alkanes to their corresponding ethylenically unsaturated carboxylic acids and higher analogues having straight or branched chains. Examples include $C_2$-$C_8$ ethylenically unsaturated carboxylic acids such as acrylic acid, methacrylic acid, butenoic acid, pentenoic acid, hexenoic acid, maleic acid, and crotonic acid. Higher analogue ethylenically unsaturated carboxylic acids are prepared from corresponding alkanes and aldehydes. For example, methacrylic acid is prepared from propane and formaldehyde. According to a separate embodiment, the corresponding acid anhydrides are also produced when preparing ethylenically unsaturated carboxylic acids from their respective alkanes. The modified catalysts of the invention are usefully employed to convert propane to arcylic acid and its higher unsaturated carboxylic acid methacrylic acid and to convert isobutane to methacrylic acid.

The modified catalysts and catalyst systems of the invention are also advantageously utilized converting alkanes to their corresponding esters of unsaturated carboxylic acids and higher analogues. Specifically, these esters include, but are not limited to, butyl acrylate from butyl alcohol and propane, β-hydroxyethyl acrylate from ethylene glycol and propane, methyl methacrylate from methanol and isobutane, butyl methacrylate from butyl alcohol and isobutane, β-hydroxyethyl methacrylate from ethylene glycol and isobutane, and methyl methacrylate from propane, formaldehyde and methanol.

In addition to these esters, other esters are formed through this invention by varying the type of alcohol introduced into the reactor and/or the alkane, alkene or alkane and alkene introduced into the reactor.

Suitable alcohols include monohydric alcohols, dihydric alcohols and polyhydric alcohols. Of the monohydric alcohols reference may be made, without limitation, to $C_1$-$C_{20}$ alcohols, preferably $C_1$-$C_6$ alcohols, most preferably $C_1$-$C_4$ alcohols. The monohydric alcohols may be aromatic, aliphatic or alicyclic; straight or branched chain; saturated or unsaturated; and primary, secondary or tertiary. Particularly preferred monohydric alcohols include methyl alcohol, ethyl alcohol, propyl alcohol, isopropyl alcohol, butyl alcohol, isobutyl alcohol and tertiary butyl alcohol. Of the dihydric alcohols reference may be made, without limitation, to $C_2$-$C_6$ diols, preferably $C_2$-$C_4$ diols. The dihydric alcohols may be aliphatic or alicyclic; straight or branched chain; and primary, secondary or tertiary. Particularly preferred dihydric alcohols include ethylene glycol (1,2-ethanediol), propylene glycol (1,2-propanediol), trimethylene glycol (1,3-propanediol), 1,2-butanediol and 2,3-butanediol. Of the polyhydric alcohols reference will only be made to glycerol (1,2,3-propanetriol).

The unsaturated carboxylic acid corresponding to the added alkane is the α,β-unsaturated carboxylic acid having the same number of carbon atoms as the starting alkane and the same carbon chain structure as the starting alkane, e.g., acrylic acid is the unsaturated carboxylic acid corresponding to propane and methacrylic acid is the unsaturated carboxylic acid corresponding to isobutane.

Similarly, the unsaturated carboxylic acid corresponding to an alkene is the α,β-unsaturated carboxylic acid having the same number of carbon atoms as the alkene and the same carbon chain structure as the alkene, e.g., acrylic acid is the unsaturated carboxylic acid corresponding to propene and methacrylic acid is the unsaturated carboxylic acid corresponding to isobutene.

Likewise, the unsaturated carboxylic acid corresponding to an unsaturated aldehyde is the α,β-unsaturated carboxylic acid having the same number of carbon atoms as the unsaturated aldehyde and the same carbon chain structure as the unsaturated aldehyde, e.g., acrylic acid is the unsaturated carboxylic acid corresponding to acrolein and methacrylic acid is the unsaturated carboxylic acid corresponding to methacrolein.

The alkene corresponding to the added alkane is the alkene having the same number of carbon atoms as the starting alkane and the same carbon chain structure as the starting alkane, e.g., propene is the alkene corresponding to propane and isobutene is the alkene corresponding to isobutane. (For alkenes having four or more carbon atoms, the double bond is in the 2-position of the carbon-carbon chain of the alkene.)

The unsaturated aldehyde corresponding to the added alkane is the α,β-unsaturated aldehyde having the same number of carbon atoms as the starting alkane and the same carbon chain structure as the starting alkane, e.g., acrolein is the unsaturated aldehyde corresponding to propane and methacrolein is the unsaturated carboxylic acid corresponding to isobutane.

Similarly, the unsaturated aldehyde corresponding to an alkene is the α,β-unsaturated carboxylic acid having the same number of carbon atoms as the alkene and the same carbon chain structure as the alkene, e.g., acrolein is the unsaturated aldehyde corresponding to propene and methacrolein is the unsaturated aldehyde corresponding to isobutene.

The modified catalysts are processed in to three-dimensional forms or are supported on three-dimensional support structures.

The support structure is three-dimensional, i.e. the support has dimensions along an x, y and z orthogonal axes of a Cartesian coordinate system, and affords a relatively high surface area per unit volume. Though lower and higher amounts are possible, in one embodiment, the support structure exhibits a surface area of 0.01 to 50 $m^2/g$, preferably 0.1 to 10 $m^2/g$.

Preferably, the support structure will have a porous structure and exhibit a pore volume percent ranging from 1 to 95%, more preferably 5 to 80%, and still more preferably 10 to 50%. Thus, the support structure permits relatively high feed velocities with insubstantial pressure drop.

Further, the support structure is sufficiently strong so that it does not fracture under the weight of the catalyst, which can range up to almost 100% of the weight of the combination of the catalyst and the support structure. More preferably, however, the support structure is at least 60% of the weight of the combination. Still more preferably, it is 70 to 99.99% of the weight of the combination. Even still more preferably, the support structure is 90 to 99.9% of the weight of the combination.

The exact physical form of the support structure is not particularly important so long as it meets the above noted general criteria. Examples of suitable physical forms of modified catalysts and supported modified catalysts include foam, honeycomb, lattice, mesh, monolith, woven fiber, non-woven fiber, gauze, perforated substrates (e.g., foil), particle compacts, fibrous mat and mixtures thereof. For these supports it will be appreciated that typically one or more open cells will be included in the structure. The cell size may vary as desired, as may the cell density, cell surface area, open frontal area and other corresponding dimensions. By way of example, one such structure has an open frontal area of at least 75%. The cell shape may also vary and may include polygonal shapes, circles, ellipses, as well as others.

The support structure may be fabricated from a material that is inert to the reaction environment of the catalytic reaction. Suitable materials include ceramics and their isomorphs such as silica, alumina (including α-, β- and γ-isomorphs), silica-alumina, aluminosilicate, zirconia, titania, boria, mullite, lithium aluminum silicate, oxide-bonded silicon carbide, metal alloy monoliths, Fricker type metal alloys, FeCrAl alloys and mixtures thereof. (Alternatively, the catalyst may be prepared so as to define the support structure itself, e.g., by "green" compacting or another suitable technique.)

The modified catalysts may be applied to the support structure using any suitable art-disclosed technique. For instance, the catalyst may be vapor deposited (e.g., by sputtering, plasma deposition or some other form of vapor deposition). The catalyst may be impregnated or coated thereon (e.g., by wash coating a support with a solution, slurry, suspension or dispersion of catalyst). The support may be coated with a catalyst powder (i.e. powder coating). (Alternatively, where the support structure is the catalyst itself, a "green" body of catalyst may be compacted to yield the desired structure.)

Modified catalysts of the invention include promoters, modifiers and oxidants. Promoters are usefully employed to oxidatively dehydrogenate alkanes to their corresponding alkenes. According to one embodiment the modified catalysts also incorporate finely dispersed metal particles including alloys (microns to nanometers) having high surface area. Alternatively, the modified catalyst is in the form of a fine gauze, including nanometer sized wires. The catalyst is impregnated on the support using techniques selected from metal sputtering, chemical vapor deposition, chemical and/or electrochemical reduction of the metal oxide.

Modifiers are usefully employed to partially oxidize alkanes to their corresponding saturated carboxylic acids and unsaturated carboxylic acids. Typical modifiers are metal oxide and MMO catalysts in the form of binary, ternary, quaternary or higher order mixed metal oxides. The modifier may preferably be present in an amount of from 0.0001 to 10 wt % of the catalyst composition (promoter plus reducible metal oxide), more preferably from 0.001 to 5 wt% of the catalyst composition, and still more preferably from 0.01 to 2 wt% of the catalyst composition.

Oxidants are usefully employed to partially oxidize alkanes, alkenes and alkanes and alkenes to their corresponding alkenes, saturated carboxylic acids and unsaturated carboxylic acids. Typically they are also metal oxide catalysts and MMO catalysts in the form of binary, ternary, quaternary or higher order mixed metal oxides. The promoter is typically present in an amount of from 0.0001 to 10 wt % of the catalyst composition (promoter plus reducible metal oxide), more preferably from 0.001 to 5 wt% of the catalyst composition, and still more preferably from 0.01 to 2 wt% of the catalyst composition. The modified catalyst is present alone or deposited, including impregnated, on the support in the form of finely dispersed metal oxide particles (microns to nanometers) having high surface area. The catalytic system

component comprises metal oxides and metal oxides used in combination with promoters in contact with a metal oxide supported.

The unmodified catalysts are prepared in steps. In a first step, a slurry or solution may be formed by admixing metal compounds, preferably at least one of which contains oxygen, and at least one solvent in appropriate amounts to form the slurry or solution. Preferably, a solution is formed at this stage of the catalyst preparation. Generally, the metal compounds contain the elements required for the particular catalyst, as previously defined.

Suitable solvents include water, alcohols including , but not limited to, methanol, ethanol, propanol, and diols, etc., as well as other polar solvents known in the art. Generally, water is preferred. The water is any water suitable for use in chemical syntheses including, without limitation, distilled water and de-ionized water. The amount of water present is preferably an amount sufficient to keep the elements substantially in solution long enough to avoid or minimize compositional and/or phase segregation during the preparation steps. Accordingly, the amount of water will vary according to the amounts and solubilities of the materials combined. However, as stated above, the amount of water is preferably sufficient to ensure an aqueous solution is formed at the time of mixing.

For example, when a mixed metal oxide of the formula $Mo_aV_bTe_cNb_dO_e$ is to be prepared, an aqueous solution of telluric acid, an aqueous solution of niobium oxalate and a solution or slurry of ammonium paramolybdate may be sequentially added to an aqueous solution containing a predetermined amount of ammonium metavanadate, so that the atomic ratio of the respective metal elements would be in the prescribed proportions.

Once the aqueous slurry or solution (preferably a solution) is formed, the water is removed by any suitable method, known in the art, to form a catalyst precursor. Such methods include, without limitation, vacuum drying, freeze drying, spray drying, rotary evaporation and air drying. Vacuum drying is generally performed at pressures ranging from 10mmHg to 500mmHg. Freeze drying typically entails freezing the slurry or solution, using , for instance, liquid nitrogen, and drying the frozen slurry or solution under vacuum. Spray drying is generally performed under an inert atmosphere such as nitrogen or argon, with an inlet temperature ranging from 125°C to 200°C and an outlet temperature ranging from 75°C to 150°C. Rotary evaporation is generally performed at a bath temperature of from 25°C to 90°C and at a pressure of from 10mmHg to 760mmHg, preferably at a bath temperature of from 40° to 90°C and at a pressure of from 10mmHg to 350mmHg, more preferably at a bath temperature of from 40°C to 60°C and at a pressure of from 10mmHg to 40mmHg. Air drying may be effected at temperatures ranging from 25°C to 90°C. Rotary evaporation or air drying are generally employed.

Once obtained, the catalyst precursor is calcined. The calcination is usually conducted in an oxidizing atmosphere, but it is also possible to conduct the calcination in a non-oxidizing atmosphere, e.g., in an inert atmosphere or in vacuo. The inert atmosphere may be any material which is substantially inert, i.e., does not react or interact with, the catalyst precursor. Suitable examples include, without limitation, nitrogen, argon, xenon, helium or mixtures thereof. Preferably, the inert atmosphere is argon or nitrogen. The inert atmosphere may flow over the surface of the catalyst precursor or may not flow thereover (a static environment). When the inert atmosphere does flow over the surface of the catalyst precursor, the flow rate can vary over a wide range, e.g., at a space velocity of from 1 to 500 $hr^{-1}$.

The calcination is usually performed at a temperature of from 350°C to 1000°C, including from 400°C to 900°C, and including from 500°C to 800°C. The calcination is performed for an amount of time suitable to form the aforementioned catalyst. Typically, the calcination is performed for from 0.5 to 30 hours, preferably from 1 to 25 hours, more preferably for from 1 to 15 hours, to obtain the desired mixed metal oxide.

In one mode of operation, the catalyst precursor is calcined in two stages. In the first stage, the catalyst precursor is calcined in an oxidizing atmosphere (e.g., air) at a temperature of from 200°C to 400°C, including from 275°C to 325°C for from 15 minutes to 8 hours, including from 1 to 3 hours. In the second stage, the material from the first stage is calcined in a non-oxidizing environment (e.g., an inert atmosphere) at a temperature of from 500°C to 900°C, including from 550°C to 800°C, for from 15 minutes to 8 hours, including from 1 to 3 hours.

Optionally, a reducing gas, such as, for example, ammonia or hydrogen, is added during the second stage calcination.

In a separate mode of operation, the catalyst precursor in the first stage is placed in the desired oxidizing atmosphere at room temperature and then raised to the first stage calcination temperature and held there for the desired first stage calcination time. The atmosphere is then replaced with the desired non-oxidizing atmosphere for the second stage calcination, the temperature is raised to the desired second stage calcination temperature and held there for the desired second stage calcination time.

Although any type of heating mechanism, e.g., a furnace, may be utilized during the calcination, it is preferred to conduct the calcination under a flow of the designated gaseous environment. Therefore, it is advantageous to conduct the calcination in a bed with continuous flow of the desired gas(es) through the bed of solid catalyst precursor particles.

With calcination, a mixed metal oxide catalyst is formed having a stoichiometric or non-stoichiometric amounts of the respective elements.

A mixed metal oxide, thus obtained, exhibits excellent catalytic activities by itself. However, the mixed metal oxide can be converted to a catalyst having higher activities by grinding.

There is no particular restriction as to the grinding method, and conventional methods may be employed. As a dry grinding method, a method of using a gas stream grinder may, for example, be mentioned wherein coarse particles are permitted to collide with one another in a high speed gas stream for grinding. The grinding may be conducted not only mechanically but also by using a mortar or the like in the case of a small scale operation.

As a wet grinding method wherein grinding is conducted in a wet state by adding water or an organic solvent to the above mixed metal oxide, a conventional method of using a rotary cylinder-type medium mill or a medium-stirring type mill, may be mentioned. The rotary cylinder-type medium mill is a wet mill of the type wherein a container for the object to be ground is rotated, and it includes, for example, a ball mill and a rod mill. The medium-stirring type mill is a wet mill of the type wherein the object to be ground, contained in a container is stirred by a stirring apparatus, and it includes, for example, a rotary screw type mill, and a rotary disc type mill.

The conditions for grinding may suitably be set to meet the nature of the above-mentioned mixed metal oxide; the viscosity, the concentration, etc. of the solvent used in the case of wet grinding; or the optimum conditions of the grinding apparatus. However, it is preferred that grinding is conducted until the average particle size of the ground catalyst precursor would usually be at most 20$\mu$m, more preferably at most 5$\mu$m. Improvement in the catalytic performance may be brought about by such grinding.

Further, in some cases, it is possible to further improve the catalytic activities by further adding a solvent to the ground catalyst precursor to form a solution or slurry, followed by drying again. There is no particular restriction as to the concentration of the solution or slurry, and it is usual to adjust the solution or slurry so that the total amount of the starting material compounds for the ground catalyst precursor is from 10 to 60 wt %. Then, this solution or slurry is dried by a method such as spray drying, freeze drying, evaporation to dryness or vacuum drying. Further, similar drying may be conducted also in the case where wet grinding is conducted.

The oxide obtained by the above-mentioned method may be used as a final catalyst, but it may further be subjected to heat treatment usually at a temperature of from 200° to 800°C for from 0.1 to 10 hours.

The mixed metal oxide thus obtained is typically used by itself as a solid catalyst, but may be formed into a catalyst together with a suitable carrier such as silica, alumina, titania, aluminosilicate, diatomaceous earth or zirconia. Further, it may be molded into a suitable shape and particle size depending upon the scale or system of the reactor. Alternatively, the metal components of the modified catalysts may be supported on materials such as alumina, silica, silica-alumina, zirconia, titania, etc. by conventional incipient wetness techniques. In one typical method, solutions containing the metals are contacted with the dry support such that the support is wetted; then, the resultant wetted material is dried, for example, at a temperature from room temperature to 200°C followed by calcination as described above. In another method, metal solutions are contacted with the support, typically in volume ratios of greater than 3 : 1 (metal solution : support), and the solution agitated such that the metal ions are ion-exchanged onto the support. The metal containing support is then dried and calcined as detailed above.

When using a catalyst system including two or more modified catalysts, the catalyst may be in the form of a physical blend of the several catalysts. Preferably, the concentration of the catalysts may be varied so that the first catalyst component will have a tendency to be concentrated at the reactor inlet while subsequent catalysts will have a tendency to be concentrated in sequential zones extending to the reactor outlet. Most preferably, the catalysts will form a layered bed (also referred to a mixed bed catalyst), with the first catalyst component forming the layer closest to the reactor inlet and the subsequent catalysts forming sequential layers to the reactor outlet. The layers abut one another or may be separated from one another by a layer of inert material or a void space.

The invention provides a process for producing an unsaturated carboxylic acid, which comprises subjecting an alkane, alkene or a mixture of an alkane and an alkene ("alkane/alkene"), to a vapor phase catalytic oxidation reaction in the presence of a catalyst containing the above promoted mixed metal oxide, to produce an unsaturated carboxylic acid.

In the production of such an unsaturated carboxylic acid, it is preferred to employ a starting material gas that contains steam. In such a case, as a starting material gas to be supplied to the reaction system, a gas mixture comprising a steam-containing alkane, or a steam-containing mixture of alkane and alkene, and an oxygen-containing gas, is usually used. However, the steam-containing alkane, or the steam-containing mixture of alkane and alkene, and the oxygen-containing gas may be alternately supplied to the reaction system. The steam to be employed may be present in the form of steam gas in the reaction system, and the manner of its introduction is not particularly limited.

Further, as a diluting gas, an inert gas such as nitrogen, argon or helium may be supplied. The molar ratio (alkane or mixture of alkane and alkene) : (oxygen) : (diluting gas) : ($H_2O$) in the starting material gas is preferably (1) : (0.1 to 10): (0 to 20) : (0.2 to 70), more preferably (1) : (1 to 5.0) : (0 to 10) : (5 to 40).

When steam is supplied together with the alkane, or the mixture of alkane and alkene, as starting material gas, the selectivity for an unsaturated carboxylic acid is distinctly improved, and the unsaturated carboxylic acid can be obtained from the alkane, or mixture of alkane and alkene, in good yield simply by contacting in one stage. However, the conventional technique utilizes a diluting gas such as nitrogen, argon or helium for the purpose of diluting the starting material. As such a diluting gas, to adjust the space velocity, the oxygen partial pressure and the steam partial pressure, an inert gas such as nitrogen, argon or helium may be used together with the steam.

As the starting material alkane it is preferred to employ a $C_{2-8}$ alkane, particularly propane, isobutane or n-butane; more preferably, propane or isobutane; most preferably, propane. According to the present invention, from such an alkane, an unsaturated carboxylic acid such as an α,β-unsaturated carboxylic acid can be obtained in good yield. For example, when propane or isobutane is used as the starting material alkane, acrylic acid or methacrylic acid will be obtained, respectively, in good yield.

In the present invention, as the starting material mixture of alkane and alkene, it is preferred to employ a mixture of $C_{2-8}$ alkane and $C_{2-8}$ alkene, particularly propane and propene, isobutane and isobutene or n-butane and n-butene. As the starting material mixture of alkane and alkene, propane and propene or isobutane and isobutene are more preferred. Most preferred is a mixture of propane and propene. According to the present invention, from such a mixture of an alkane and an alkene, an unsaturated carboxylic acid such as an α,β-unsaturated carboxylic acid can be obtained in good yield. For example, when propane and propene or isobutane and isobutene are used as the starting material mixture of alkane and alkene, acrylic acid or methacrylic acid will be obtained, respectively, in good yield. Preferably, in the mixture of alkane and alkene, the alkene is present in an amount of at least 0.5% by weight, more preferably at least 1.0% by weight to 95% by weight; most preferably, 3% by weight to 90% by weight.

As an alternative, an alkanol, such as isobutanol, which will dehydrate under the reaction conditions to form its corresponding alkene, i.e. isobutene, may also be used as a feed to the present process or in conjunction with the previously mentioned feed streams.

The purity of the starting material alkane is not particularly limited, and an alkane containing a lower alkane such as methane or ethane, air or carbon dioxide, as impurities, may be used without any particular problem. Further, the starting material alkane may be a mixture of various alkanes. Similarly, the purity of the starting material mixture of alkane and alkene is not particularly limited, and a mixture of alkane and alkene containing a lower alkene such as ethene, a lower alkane such as methane or ethane, air or carbon dioxide, as impurities, may be used without any particular problem. Further, the starting material mixture of alkane and alkene may be a mixture of various alkanes and alkenes.

There is no limitation on the source of the alkene. It may be purchased, per se, or in admixture with an alkane and/or other impurities. Alternatively, it can be obtained as a by-product of alkane oxidation. Similarly, there is no limitation on the source of the alkane. It may be purchased, per se, or in admixture with an alkene and/or other impurities. Moreover, the alkane, regardless of source, and the alkene, regardless of source, may be blended as desired.

The detailed mechanism of the oxidation reaction of the present invention is not clearly understood, but the oxidation reaction is carried out by oxygen atoms present in the above mixed metal oxide or by molecular oxygen present in the feed gas. To incorporate molecular oxygen into the feed gas, such molecular oxygen may be pure oxygen gas. However, it is usually more economical to use an oxygen-containing gas such as air, since purity is not particularly required.

It is also possible to use only an alkane, or a mixture of alkane and alkene, substantially in the absence of molecular oxygen for the vapor phase catalytic reaction. In such a case, it is preferred to adopt a method wherein a part of the catalyst is appropriately withdrawn from the reaction zone from time to time, then sent to an oxidation regenerator, regenerated and then returned to the reaction zone for reuse. As the regeneration method of the catalyst, a method may, for example, be mentioned which comprises contacting an oxidative gas such as oxygen, air or nitrogen monoxide with the catalyst in the regenerator usually at a temperature of from 300° to 600°C.

This aspect present invention is described in still further detail with respect to a case where propane is used as the starting material alkane and air is used as the oxygen source. The reaction system may be preferably a fixed bed system. The proportion of air to be supplied to the reaction system is important for the selectivity for the resulting acrylic acid, and it is usually at most 25 moles, preferably from 0.2 to 18 moles per mole of propane, whereby high selectivity for acrylic acid can be obtained. This reaction can be conducted usually under atmospheric pressure, but may be conducted under a slightly elevated pressure or slightly reduced pressure. With respect to other alkanes such as isobutane, or to mixtures of alkanes and alkenes such as propane and propene, the composition of the feed gas may be selected in accordance with the conditions for propane.

Typical reaction conditions for the oxidation of propane or isobutane to acrylic acid or methacrylic acid may be utilized in the practice of the present invention. The process may be practiced in a single pass mode (only fresh feed is fed to the reactor) or in a recycle mode (at least a portion of the reactor effluent is returned to the reactor). General conditions for the process of the present invention are as follows: the reaction temperature can vary from 200°C to 700°C, but is usually in the range of from 200°C to 550°C, more preferably 250°C to 480°C, most preferably 300°C to 400°C; the gas space velocity, SV, in the vapor phase reaction is usually within a range of from 100 to 10,000 $hr^{-1}$, preferably 300 to 6,000 $hr^{-1}$, more preferably 300 to 2,000 $hr^{-1}$; the average contact time with the catalyst can be from 0.01 to 10 seconds or more, but is usually in the range of from 0.1 to 10 seconds, preferably from 0.2 to 6 seconds; the pressure in the reaction zone usually ranges from 0 to 75 psig, but is preferably no more than 50 psig. In a single pass mode process, it is preferred that the oxygen be supplied from an oxygen-containing gas such as air. The single pass mode process may also be practiced with oxygen addition. In the practice of the recycle mode process, oxygen gas by

itself is the preferred source so as to avoid the build up of inert gases in the reaction zone. The feed of hydrocarbon in the catalytic process is dependent on the mode of operation (e.g. single pass, batch, recycle, etc.) and ranges from 2 wt. % to 50 wt. %. According to a separate embodiment, the catalytic process is a batch process. According to a separate process, the catalytic process is run continuously. The catalytic process all conventional beds including, but not limited to static fluid beds, fluidized beds, moving beds, transport beds, moving beds such as rising and ebulating beds. Any catalytic process is carried out under steady state conditions or non steady state conditions.

Of course, in the oxidation reaction of the present invention, it is important that the hydrocarbon and oxygen concentrations in the feed gases be maintained at the appropriate levels to minimize or avoid entering a flammable regime within the reaction zone or especially at the outlet of the reactor zone. Generally, it is preferred that the outlet oxygen levels be low to both minimize after-burning and, particularly, in the recycle mode of operation, to minimize the amount of oxygen in the recycled gaseous effluent stream. In addition, operation of the reaction at a low temperature (below 450°C) is extremely attractive because after-burning becomes less of a problem which enables the attainment of higher selectivity to the desired products. The catalyst of the present invention operates more efficiently at the lower temperature range set forth above, significantly reducing the formation of acetic acid and carbon oxides, and increasing selectivity to acrylic acid. As a diluting gas to adjust the space velocity and the oxygen partial pressure, an inert gas such as nitrogen, argon or helium may be employed.

When the oxidation reaction of propane, and especially the oxidation reaction of propane and propene, is conducted by the method of the present invention, carbon monoxide, carbon dioxide, acetic acid, etc. may be produced as by-products, in addition to acrylic acid. Further, in the method of the present invention, an unsaturated aldehyde may sometimes be formed depending upon the reaction conditions. For example, when propane is present in the starting material mixture, acrolein may be formed; and when isobutane is present in the starting material mixture, methacrolein may be formed. In such a case, such an unsaturated aldehyde can be converted to the desired unsaturated carboxylic acid by subjecting it again to the vapor phase catalytic oxidation with the promoted mixed metal oxide-containing catalyst of the present invention or by subjecting it to a vapor phase catalytic oxidation reaction with a conventional oxidation reaction catalyst for an unsaturated aldehyde.

Turning now in more specific detail to another aspect of the present invention, the method comprises subjecting an alkane, or a mixture of an alkane and an alkene, to a vapor phase catalytic oxidation reaction with ammonia in the presence of a catalyst containing the above mixed metal oxide, to produce an unsaturated nitrile.

In the production of such an unsaturated nitrile, as the starting material alkane, it is preferred to employ a $C_{2-8}$ alkane such as propane, butane, isobutane, pentane, hexane and heptane. However, in view of the industrial application of nitriles to be produced, it is preferred to employ a lower alkane having 3 or 4 carbon atoms, particularly propane and isobutane.

Similarly, as the starting material mixture of alkane and alkene, it is preferred to employ a mixture of $C_{2-8}$ alkane and $C_{2-8}$ alkene such as propane and propene, butane and butene, isobutane and isobutene, pentane and pentene, hexane and hexene, and heptane and heptene. However, in view of the industrial application of nitriles to be produced, it is more preferred to employ a mixture of a lower alkane having 3 or 4 carbon atoms and a lower alkene having 3 or 4 carbon atoms, particularly propane and propene or isobutane and isobutene. Preferably, in the mixture of alkane and alkene, the alkene is present in an amount of at least 0.5% by weight, more preferably at least 1.0% by weight to 95% by weight, most preferably 3% by weight to 90% by weight.

The purity of the starting material alkane is not particularly limited, and an alkane containing a lower alkane such as methane or ethane, air or carbon dioxide, as impurities, may be used without any particular problem. Further, the starting material alkane may be a mixture of various alkanes. Similarly, the purity of the starting material mixture of alkane and alkene is not particularly limited, and a mixture of alkane and alkene containing a lower alkene such as ethene, a lower alkane such as methane or ethane, air or carbon dioxide, as impurities, may be used without any particular problem. Further, the starting material mixture of alkane and alkene may be a mixture of various alkanes and alkenes.

There is no limitation on the source of the alkene. It may be purchased, per se, or in admixture with an alkane and/or other impurities. Alternatively, it can be obtained as a by-product of alkane oxidation. Similarly, there is no limitation on the source of the alkane. It may be purchased, per se, or in admixture with an alkene and/or other impurities. Moreover, the alkane, regardless of source, and the alkene, regardless of source, may be blended as desired.

Accoording to a separate embodiment, a short contact reactor is employed with the one or more modified catalysts of the invention. The short contact time reactor is of a type suitable for the use of a fixed catalyst bed in contact with a gaseous stream of reactants. For instance, a shell and tube type of reactor may be utilized, wherein one or more tubes are packed with catalyst(s) so as to allow a reactant gas stream to be passed in one end of the tube(s) and a product stream to be withdrawn from the other end of the tube(s). The tube(s) being disposed in a shell so that a heat transfer medium may be circulated about the tube(s).

In the case of the utilization of a single catalyst or catalyst system, the gas stream comprising the alkane, molecular oxygen and any additional reactant feeds including but not limited to alkenes, oxygen, air, hydrogen, carbon

monoxide, carbon dioxide, formaldehyde and alcohols, steam and any diluents including nitrogen, argon may all be fed into the front end(s) of the tube(s) together. Alternatively, the alkane and the molecular oxygen-containing gas may be fed into the front end(s) of the tube(s) while the additional reactants, steam and diluents may be fed (also referred to as staging) into the tube(s) at a predetermined downstream location (typically chosen so as to have a certain minimum concentration of product alkene present in the gas stream passing through the tube(s), e.g., 3%, preferably 5%, most preferably 7%).

In the case of the utilization of catalyst systems including two or more catalysts, e.g., a first catalyst component and a second catalyst component as described above, once again the gas stream comprising the alkane, the oxygen-containing gas and any additional reactant feeds including but not limited to alkenes, oxygen, air, hydrogen, carbon monoxide, carbon dioxide, formaldehyde and alcohols, steam and any diluents including nitrogen, argon are fed to the front end(s) of the tube(s) together. Alternatively, and preferably, the alkane and the molecular oxygen-containing gas are staged into the front end(s) of the tube(s) while any additional reactant feeds, steam and diluents are staged into the tube(s) at a predetermined downstream location (typically chosen so at have a certain minimum concentration of desired product present in the gas stream passing through the tube(s),as set forth above; or in the case of the utilization of layered beds of catalyst, as described above, intermediate two layered catalyst beds).

Typical reaction conditions for the oxidation of propane or isobutane to acrylic acid or methacrylic acid including respective esters thereof which are utilized in the practice of the present invention include: reaction temperatures which can vary from 300°C to 1000°C, but are usually in the range of flame temperatures (from 500°C to 1000°C); the average contact time with the catalyst (i.e. the reactor residence time) is not more than 100 milliseconds, including not more than 80 milliseconds, and including not more than 50 milliseconds; the pressure in the reaction zone usually ranges from 0 to 75 psig, including no more than 50 psig.

The invention provides a process for preparing unsaturated carboxylic acids from corresponding alkanes, the process comprising the step of: providing one or more modified catalysts cumulatively effective at converting the gaseous alkane to its corresponding gaseous unsaturated carboxylic acid;

wherein the second catalyst layer is separated at a distance downstream from the first catalyst layer and the reactor is operated at a temperature of from 100°C to 700°C, with a reactor residence time of no less than 100 milliseconds. As a separate embodiment, a short contact time reactor is operated at a temperature of from 100°C to 700°C, with a reactor residence time of than 100 or less milliseconds.

300°C to 400°C, with a second reaction zone residence time of no greater than 100 milliseconds;

It is preferred to pass a gaseous stream comprising propane or isobutane and molecular oxygen to the reactor. In addition, the feed may contain an additional reactant, adjuvant such as steam or a diluent such as an inert gas, e.g., nitrogen, argon or carbon dioxide.

In a separate embodiment, the gaseous stream of the alkane is passed through the reactor in a single pass or wherein any unreacted alkane is recycled back into the gaseous stream of alkane entering the reactor and any saturated carboxylic acid is recycled back into the second catalyst zone to increase the overall yield of unsaturated carboxylic acid.

The invention also provides a process comprising the steps of: (a) converting an alkane to its corresponding products selected from alkene, unsaturated carboxylic acid, and higher analogue unsaturated carboxylic acid in a short contact time reactor using the catalyst systems of the invention; and (b) adding the resulting product or products to the front end of a second fixed bed oxidation reactor with the product(s) from the first reactor acting as feed to the second reactor. For example, propane is converted to propylene using a catalyst system as described in a short contact time reactor. The propylene is then fed to second oxidation reactor that converts its to acrylic acid. According to one embodiment this includes feeding any unreacted alkane from the first reactor to the second reactor to recycle the alkane. For example, any unreacted propane is recycled to the first SCTR or added as a feed to the second oxidation reactor. The second oxidation reactor comprises any conventional industrial scale oxidation reactor used for converting alkenes to unsaturated carboxylic acids at longer residence times (seconds). Alternatively, the second oxidation reactor comprises a second SCTR operating at millisecond residence times.

Any source of molecular oxygen may be employed in this process, e.g., oxygen, oxygen-enriched gases or air. Air may be the most economical source of oxygen, especially in the absence of any recycle.

The invention also provides a process for the production of esters of unsaturated carboxylic acids, the process comprising the step of:

passing a gaseous alkane, molecular oxygen and a gaseous alcohol to a short contact time reactor, the reactor including a mixed catalyst bed comprising (a) a first catalyst layer comprising one or more modified catalysts cumulatively effective at converting the gaseous alkane to its corresponding gaseous unsaturated carboxylic acid; wherein the catalysts of the first layer are impregnated on a metal oxide support; and (b) a second catalyst layer comprising one or more unmodified or modified catalysts cumulatively effective at converting the gaseous unsaturated carboxylic acid to its corresponding gaseous ester;

wherein the second catalyst layer is separated at a distance downstream from the first catalyst layer and the reactor is operated at a one or more temperatures of from 100°C to 1000°C. In a separate embodiment the modified catalysts are

partitioned into one or more zones, the first reaction zone being operated at a temperature of from 100°C to 1000°C, the second reaction zone being operated at a temperature of from 300°C to 400°C. The second catalyst comprises one or more unmodified or modified superacids.

According to yet another embodiment, provides a process for catalytically converting alkanes to their corresponding higher unsaturated carboxylic acids and then catalytically converting them to their corresponding esters in the presence of specific alcohols.

The second catalyst comprises one or more modified or umodified superacid. A superacid, according to the definition of Gillespie, is an acid that is stronger than 100% sulfuric acid, i.e. it has a Hammett acidity value $H_0$ < -12. Representative superacids include, but are not limited to: zeolite supported $TiO_2/(SO_4)_2$, $(SO_4)_2/ZrO_2$-$TiO_2$, $(SO_4)_2/ZrO_2$-$Dy_2O_3$, $(SO_4)_2/TiO_2$, $(SO_4)_2/ZrO_2$-NiO, $SO_4/ZrO_2$, $SO_4/ZrO_2Al_2O_3$, $(SO_4)_2/Fe_2O_3$, $(SO_4)_2/ZrO_2$, $C_4F_9SO_3H$-$SbF_5$, $CF_3SO_3H$-$SbF_5$, Pt/sulfated zirconium oxide, $HSO_3F$-$SO_2ClF$, $SbF_5$-$HSO_3F$-$SO_2ClF$, $MF_5/AlF_3$ (M=Ta, Nb, Sb), $B(OSO_2CF_3)_3$, $B(OSO_2CF_3)_3$-$CF_3SO_3H$, $SbF_5$-$SiO_2$-$Al_2O_3$, $SbF_5$-$TiO_2$-$SiO_2$ and $SbF_5$-$TiO_2$. Preferably, solid superacids are utilized, e.g., sulfated oxides, supported Lewis acids and supported liquid superacids. Only a small number of oxides produce superacid sites on sulfation, including $ZrO_2$, $TiO_2$, $HfO_2$, $Fe_2O_3$ and $SnO_2$. The acid sites are generated by treating an amorphous oxyhydrate of these elements with $H_2SO_4$ or $(NH_4)_2SO_4$ and calcining the products at temperatures of 500°C - 650°C. During the calcination, the oxides are transformed into a crystalline tetragonal phase, which is covered by a small number of sulfate groups. $H_2MoO_4$ or $H_2WO_4$ may also be used to activate the oxide.

In a separate embodiment of the present invention, an alcohol is reacted with an unsaturated aldehyde to form an acetal. Such reaction can be carried out by contacting the aldehyde with an excess of the anhydrous alcohol in the presence of a small amount of an anhydrous acid, e.g., anhydrous HCl. Preferably, the aldehyde and the alcohol can be passed through a bed containing an acid catalyst, e.g., through a bed of a strongly acidic ion exchange resin, such as Amberlyst 15.

The so-formed acetal and molecular oxygen are fed to a reactor containing at least one catalyst effective for the oxidation of the acetal to its corresponding ester. Examples of such a catalyst include well known Pd and Bi on alumina or V oxides.

The modified catalysts of the invention are usefully employed in catalytic processes described in a pending provisional U. S. Application (Ser. No. 06/000000). The application provides a process which addresses the problem of a decreased total yield of oxidation product in multi-stage vapor phase oxidation reactions which employ staged oxygen arrangements for conversion of lower alkanes and alkenes, and mixtures thereof, to unsaturated carboxylic acids and/or unsaturated nitriles. More particularly, it has been discovered that in such processes, the removal of at least a portion of the oxidation product from each intermediate effluent stream, for example, by inter-stage partial condensation, prior to adding more oxygen and feeding the effluent stream to the next stage, unexpectedly results in overall cumulative oxidation product yields greater than either the original single-stage system or the system including only staged oxygen arrangements.

The present invention provides an improved process for the production of unsaturated carboxylic acids and unsaturated nitriles from their corresponding $C_2$-$C_8$ alkanes, or mixtures of $C_2$-$C_8$ alkanes and alkenes, that utilizes a multi-stage reaction system and includes the steps of separating the oxidation product from one or more intermediate effluent streams, as well as feeding additional oxygen to reaction zones subsequent to the first reaction zone.

The process using modified catalysts of the invention is for producing unsaturated carboxylic acids or unsaturated nitriles by vapor phase oxidation reaction of their corresponding $C_2$-$C_8$ alkanes, $C_2$-$C_8$ alkenes, and mixtures thereof. The process of the present invention uses a reaction system, having at least two reaction zones arranged in series with one another and at least one catalyst capable of catalyzing the vapor phase oxidation reaction disposed in each of the at least two reaction zones. Furthermore, at least one intermediate effluent stream exits a preceding one of the at least two reaction zones and is at least partially fed to a subsequent one of the at least two reaction zones. The process of the present invention comprises separating the at least one intermediate effluent stream into at least an intermediate product stream comprising an oxidation product selected from the group consisting of an unsaturated carboxylic acid and an unsaturated nitrile, and an intermediate feed stream comprising starting materials selected from the group consisting of an unreacted $C_2$-$C_8$ alkane, an unreacted $C_2$-$C_8$. alkene, and mixtures thereof; feeding the intermediate feed stream to the subsequent reaction zone; and feeding an oxygen-containing gas to the subsequent reaction zone. In one alternative embodiment, two or more of the reaction zones may be contained within a single reactor vessel.

The separating step may be performed by cooling the at least one intermediate effluent stream such that at least a portion of the oxidation products condenses out of the at least one intermediate effluent stream. Such cooling may be achieved with a condenser. The separating step may, alternatively, be performed using an absorber.

In a particular application of the present invention, the $C_2$-$C_8$ alkane, $C_2$-$C_8$ alkene, or mixture thereof may comprise propane, propene, or a mixture thereof, and the oxidation product may comprise acrylic acid.

The process also comprises feeding ammonia-containing gas to each of the at least two reaction zones. In a particular application of the process, in which ammonia-containing gas is fed to each of the at least two reaction zones,

the $C_2$-$C_8$ alkane, $C_2$-$C_8$ alkene, or mixture thereof, may comprise propane, propene, or a mixture thereof, and the oxidation product may comprise acrylonitrile.

Separators suitable for use with the present invention include any suitable fluid separator capable of separating a gaseous product stream into multiple streams according to composition, such as separating a gaseous output stream into a first stream containing primarily the desired reaction product(s) and a second stream containing primarily unreacted materials and by-products. For example, while not intending to be limited, the separator may be a partial condenser 16, 20, such as a conventional heat exchanger, capable of cooling the gaseous output stream sufficiently to condense and separate out at least a portion of the lowest boiling point components of the gaseous output stream would be suitable for use with the process 10 of the present invention. The coolant in such a condenser may be, for example, without limitation, cooling tower water having a temperature between 85°F and 105°F (29°C to 40°C), or chilled water having a temperature between 32°F and 40°F (0°C and 5°C). In addition, for example, the separators may include gas absorbers or gas adsorbers.

Suitable starting materials, which are discussed hereinafter and which are readily determinable by persons having ordinary skill in the art, are fed into the first reaction zone 12. In the first reaction zone 12, the starting materials come into contact with the catalyst and react with one another to form the desired oxidation products, as well as various side products and by-products, according to the particular types of $C_2$ to $C_5$8alkanes and alkenes used.

Suitable starting materials for the process 10 of the present invention depend upon the desired oxidation product and typically include, but are not limited to, a $C_2$ to $C_8$ alkane, a $C_2$ to $C_8$ alkene, or a mixture thereof, and an oxygen-containing gas, as well as, optionally, steam, diluting gases and ammonia. The starting materials may be added separately and simultaneously to the first reaction zone 12, or they may be mixed and fed to the first reaction zone 12 as one or more combined streams. For example, as explained in further detail hereinafter, the initial feed stream 22, shown in Figure 1, may be a combined stream comprising an oxygen-containing gas and a $C_2$ to $C_8$ alkane, a $C_2$ to $C_8$ alkene, or a mixture thereof. The optional supplemental streams 24, 24', 24", shown in phantom in Figure 1, may be, for example, steam-containing gases or ammonia-containing gases, depending upon the particular oxidation products desired. The optional supplemental streams 24, 24', 24" may even comprise additional $C_2$ to $C_8$ alkane, $C_2$ to $C_8$ alkene, or a mixture thereof.

The detailed mechanism of the oxidation reaction of the present invention is not clearly understood, but the oxidation reaction is carried out by oxygen atoms present in the above mixed metal oxide or by molecular oxygen present in the feed gas. Addition of oxygen-containing gas to the starting materials provides such molecular oxygen to the reaction system. The term "oxygen-containing gas," as used herein, refers to any gas comprising from 0.01% up to 100% oxygen, including, for example, air. Thus, although the oxygen-containing gas may be pure oxygen gas, it is usually more economical to use an oxygen-containing gas such as air, since purity is not particularly required.

The purity of the starting material, i.e., the $C_2$ to $C_8$ alkane, the $C_2$ to $C_8$ alkene, or the mixture thereof, is not particularly limited. Thus, commercial grades of such alkanes, or mixtures of such alkanes and alkenes, may be used as starting material for the process 10 of the present invention, although higher purities are advantageous from the standpoint of minimizing competing side reactions. In addition, mixed $C_2$ to $C_8$ alkane/alkene feeds are generally more easily obtained and may include price incentives (e.g., lower separation costs) relative to pure $C_2$ to $C_8$ alkane feeds. For example, a mixture of alkane and alkene containing a lower alkene such as ethene, a lower alkane such as methane or ethane, air or carbon dioxide, as impurities, may be used without any particular problem. Further, the starting material mixture of $C_2$ to $C_8$ alkane and alkene may be a mixture of various $C_2$ to $C_8$ alkanes and alkenes. Further details concerning the starting materials will be discussed hereinafter in connection with particular embodiments of the present invention.

Suitable diluting gases include, but are not limited to, one or more of: carbon monoxide, carbon dioxide, or mixtures thereof, an inert gas, such as nitrogen, argon, helium, or mixtures thereof. A suitable molar ratio of the starting materials for the initial feed stream 22, ($C_2$ to $C_8$ alkane, $C_2$ to $C_8$ alkene, or a mixture thereof) (oxygen) : (diluting gas) : ($H_2O$), would be, for example, (1) : (0.1 to 10) : (0 to 20) : (0.2 to 70), for example, including but not limited to, (1) : (1 to 5.0) : (0 to 10) : (5 to 40).

Where it is desired to produce unsaturated carboxylic acids, it is beneficial to include steam among the starting materials. In such a case, for example, a gaseous input stream comprising a mixture of and oxygen-containing gas and a steam-containing $C_2$ to $C_8$ alkane, or a steam-containing $C_2$ to $C_8$ alkene, or a steam-containing mixture thereof, may be used. It is noted that the steam may be added to the first reaction zone separately from the $C_2$ to $C_8$ alkane, the $C_2$ to $C_8$ alkene, or the mixture thereof, and the oxygen-containing gas, as an initial feed stream and an optional steam stream, respectively.

In accordance with the process, at least a portion of the one or more oxidation products is separated from the first effluent stream, for example, by using a separator, such as the condenser, to produce an intermediate product stream and an intermediate feed stream. The intermediate product stream typically contains, but is not limited to, at least a portion of the one or more oxidation products from the first effluent stream, as well as other condensables, such as organic acids, aldehydes, ketones, and water. The intermediate product stream may be fed to additional processing

apparatus (not shown) to undergo further separation and purification processes. The intermediate feed stream contains, but is not limited to, at least a portion of the unreacted oxygen, unreacted $C_2$ to $C_8$ alkane or alkene, or mixture thereof, and possibly reaction by-products such as acetic acid and carbon dioxide, and, possibly, unreacted water and unreacted ammonia, depending upon the starting materials used.

The cumulative yield of the desired oxidation product produced by the above-described process is greater than the cumulative yield of the desired oxidation product that is produced by a process that does not include both separating at least a portion of the one or more oxidation products from the first effluent stream, as well as feeding additional oxygen-containing gas to the second reaction zone. In addition, the cumulative yield of the one or more oxidation products produced by the above-described process is greater than the cumulative yield of the one or more oxidation products that is produced by a process that includes only feeding additional oxygen-containing gas to the second reaction zone, without separating at least a portion of the one or more oxidation products from the first effluent stream. The process allows for the use of starting materials containing a higher concentration of the $C_2$ to $C_8$ alkane, the $C_2$ to $C_8$ alkene, or mixture thereof. It is also believed that a greater portion of the oxygen in each subsequent reaction remains available for reacting and converting the $C_2$ to $C_8$ alkanes and alkenes.

The purity of the starting material alkene is not limited, and an alkene containing a lower alkene such as ethene, air or carbon dioxide, as impurities, may be used without any particular problem. Further, the starting material alkene may be a mixture of various alkenes. Similarly, the purity of the starting material mixture of alkene and alkane is not particularly limited, and a mixture of alkene and alkane containing a lower alkene such as ethene, a lower alkane such as methane or ethane, air or carbon dioxide, as impurities, may be used without any particular problem. Further, the starting material mixture of alkene and alkane may be a mixture of various alkenes and alkanes.

There is no limitation on the source of the alkene. It may be purchased, per se, or in admixture with an alkane and/or other impurities. Alternatively, it can be obtained as a by-product of alkane oxidation. Similarly, there is no limitation on the source of the alkane. It may be purchased, per se, or in admixture with an alkene and/or other impurities. Moreover, the alkane, regardless of source, and the alkene, regardless of source, may be blended as desired.

The detailed mechanism of the oxidation reaction of this embodiment of the present invention is not clearly understood. When it is desired to incorporate molecular oxygen in the starting materials, the oxygen-containing gas may be pure oxygen gas. However, since high purity is not required, it is usually economical to use air as the oxygen-containing gas. The following illustrative examples are provided to further demonstrate the utility of the present invention and are not in any way construed to be limiting. Moreover, the examples provided are representative examples that broadly enable the claimed scope of the invention. In the following Examples, "propane conversion" is synonymous with "feed conversion" and was calculated in accordance with the formulas provided earlier hereinabove. Furthermore, "AA yield" means acrylic acid yield and is synonymous with "product yield" and was calculated in accordance with the formulas provided earlier hereinabove.

Unless otherwise specified, all percentages recited in the following Examples are by volume, based on the total volume of the feed or product gas stream.

**Examples**

Conversion of propane to acrylic acid by single-step catalytic vapor phase oxidation was performed utilizing varying amounts of carbon dioxide in the starting materials, from 0 vol% to 50 vol%, in 10% increments, and at varying temperatures between 365°C to 390°C, in increments of 5°C. The amount of propane in the starting materials was kept constant at 9.2 vol% and the amount of oxygen in the starting materials was kept constant at 19.1 vol%, based upon the total volume of the starting materials fed to the reaction zones, with the balance comprising argon as a diluting gas. All processes were operated at atmospheric pressure (i.e., 1 atmosphere).

Each of the examples was performed in an experimental reactor system using a three-zone tube reactor configuration at normal and vacuum conditions. This reaction system comprised three basic components: a valve manifold, a reactor and a mass spectrometer. The mass spectrometer is contained in a high-vacuum system that can easily accommodate low-intensity fast transient response experiments, and can handle high volume continuous flows as a result of a specially designed slide valve that permits the reactor to operate at vacuum or high pressure conditions ($10^{-8}$ to 7000 torr).

The reactor tube length was 33 millimeters ("mm") and its diameter was 5 mm. The three reaction zones included two inert zones, each of 12 mm in length and packed with 730 mg of quartz particles, and a one catalyst zone, positioned between the inert zones. The catalyst zone was 3.3 mm in length and packed with 120 mg of a suitable catalyst.

The starting material gas mixtures of propane, oxygen, $CO_2$, and argon were passed through a fritted, heated water bubbler (at 65°C) before being admitted to the reactor through a continuous flow valve at 1 atmosphere. Additional reaction conditions included a contact time of 3.3 seconds, and at each catalyst bed temperature, the heating rates were varied from 0.5°C/minute to 20°C/minute.

For each process example, the reactor was evacuated to $10^{-6}$ torr and small reactant or product gas pulses

($10^{13}$ molecules/pulse) were passed over the catalyst. The outlet (Le.: product) composition measurements were performed by passing a small portion of the outlet flow into the mass spectrometer chamber through a needle valve located between the reactor exit and a vacuum chamber.

The catalyst used in the examples was prepared in a manner similar to the synthesis procedure disclosed in U.S. Patent No. 6,642,174. More particularly, a catalyst of nominal composition $Mo_{1.0}V_{0.3}Te_{0.23}Nb_{0.17}O_x$ was prepared in the presence of nitric acid in the following manner: 200 mL of an aqueous solution containing ammonium heptamolybdate tetrahydrate (1.0 M Mo), ammonium metavanadate (0.3 M V) and telluric acid (0.23M Te) formed by dissolving the corresponding salts in water at 70°C, was added to a 2000mL rotavap flask. Then 200 mL of an aqueous solution of ammonium niobium oxalate (0.17 M Nb), oxalic acid (0.155 M) and nitric acid (0.24 M) were added thereto. After removing the water via a rotary evaporator with a warm water bath at 50°C and 28mm Hg, the solid materials were further dried in a vacuum oven at 25°C overnight and then calcined.

Calcination was effected by placing the solid materials in an air atmosphere and then heating them to 275°C at 10°C/min and holding them under the air atmosphere at 275°C for one hour; the atmosphere was then changed to argon and the material was heated from 275°C to 600°C at 2°C/min and the material was held under the argon atmosphere at 600°C for two hours. XRD analysis revealed diffraction peaks at the following angles (±0.3°) of 2θ: 22.1°, 36.2°, 45.2° and 50.0°.

**Example Set A**

The reaction temperature was held constant at about 365°C and the amount of carbon dioxide was varied from 0 vol% to 50 vol%, in 10 vol% increments. The results are shown in Table 1 below and the graph provided in Figure 1.

**Example Set B**

The reaction temperature was held constant at about 370°C and the amount of carbon dioxide was varied from 20 vol% to 50 vol%, in 10 vol% increments. The results are shown in Table 1 below and the graph provided in Figure 1.

**Example Set C**

The reaction temperature was held constant at about 375°C and the amount of carbon dioxide was varied from 0 vol% to 50 vol%, in 10 vol% increments. The results are shown in Table 1 below and the graph provided in Figure 1.

**Example Set D**

The reaction temperature was held constant at about 380°C and the amount of carbon dioxide was varied from 0 vol% to 50 vol%, in 10 vol% increments. The results are shown in Table 1 below and the graph provided in Figure 1.

Example Set E

The reaction temperature was held constant at about 385°C and the amount of carbon dioxide was varied from 0 vol% to 50 vol%, in 10 vol% increments. The results are shown in Table 1 below and the graph provided in Figure 1.

Example Set F

The reaction temperature was held constant at about 390°C and the amount of carbon dioxide was varied from 0 vol% to 50 vol%, in 10 vol% increments. The results are shown in Table 1 below and the graph provided in Figure 1.

Table 1

| %$CO_2$ in Feed | Acrylic Acid Yield (%) | | | | | |
|---|---|---|---|---|---|---|
| | Set A (365°C) | Set B (370°C) | Set C (375°C) | Set D (380°C) | Set E (385°C) | Set F (390°C) |
| | | | | | | |
| 0 | 20.8 | | 22.2 | 22.6 | 23.4 | 24.3 |
| 10 | 20.9 | | 22.5 | 23.1 | | |
| 20 | 21.5 | 22.1 | 23.9 | 24.3 | 24.7 | 25.3 |

Table continued

| $\%CO_2$ in Feed | Acrylic Acid Yield (%) | | | | | |
|---|---|---|---|---|---|---|
| | Set A (365°C) | Set B (370°C) | Set C (375°C) | Set D (380°C) | Set E (385°C) | Set F (390°C) |
| 30 | 21.9 | 22.5 | 23.7 | 24.3 | 24.7 | 25.3 |
| 40 | 22.7 | 23.1 | 24.1 | 24.9 | 25.5 | 25.6 |
| 50 | 22.1 | 22.9 | 23.8 | 24.9 | 25.8 | 26.4 |

It is noted that, since the carbon dioxide content was zero for the first data point for each Example Set (except for Set B), this point is the comparative example at each of the six operating temperatures tested. The remaining data points represent various applications of the present invention and show that, at a given temperature, increased acrylic acid yield can be achieved by increasing the amount of carbon dioxide feed to the oxidation process.

MMO1 catalyst performance is improved with a sub-monolayer deposition of Te onto its surface by vapor deposition. The selectivity to acrylic acid improved by approximately 6% and the acrylic acid yield by 3%, absolute. Applying a similar Te loading onto MMO1 by wet impregnation methods did not improve catalytic performance. Post treatment of the Te vapor deposited MMO1 catalyst with oxygen at elevated temperatures gave improved catalytic performance when compared to a corresponding sample treated with an inert gas at the same elevated temperatures.

## Claims

1. An improved (amm)oxidation catalyst comprising: one or more modified mixed metal oxide catalysts having the empirical formula:

$$M_eMoV_aNb_bX_cZ_dO_n$$

wherein $M_e$ is at least one or more chemical modifying agents, X is at least one element selected from the group consisting of Te and Sb, Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements, $0.1 \leq a \leq 1.0$, $0.01 \leq b \leq 1.0$, $0.01 \leq c \leq 1.0$, $0 \leq d \leq 1.0$ and n, e are determined by the oxidation states of the other elements; wherein the catalyst is improved with a sub-monolayer deposition of Te onto its surface by vapor deposition.

2. A surface modified (amm)oxidation catalyst comprising: one or more modified mixed metal oxide catalysts having the empirical formula:

$$M_eMoV_aNb_bX_cZ_dO_n$$

wherein $M_e$ is at least one or more chemical modifying agents, X is at least one element selected from the group consisting of Te and Sb, Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements, $0.1 \leq a \leq 1.0$, $0.01 \leq b \leq 1.0$, $0.01 \leq c \leq 1.0$, $0 \leq d \leq 1.0$ and n, e are determined by the oxidation states of the other elements; wherein the catalyst surface is modified with a sub-monolayer deposition of Te onto its surface by vapor deposition.

3. A process for preparing an improved (amm)oxidation catalyst comprising the step of: depositing one or more elements X and Z in the vapor phase,
wherein X is at least one element selected from the group consisting of Te and Sb, Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements, to one or more metals to one or more mixed metal catalysts; wherein the catalyst is improved with a sub-monolayer deposition of Te onto its surface by vapor deposition.

4. A process for modifiying the surface of one or more mixed metal oxide catalysts comprising the step of: depositing one or more elements X and Z in the vapor phase, wherein X is at least one element selected from the group consisting of Te and Sb, Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth

elements, to one or more metals to one or more mixed metal catalysts; wherein the catalyst surface is modified with a sub-monolayer deposition of Te onto its surface by vapor deposition.

**5.** A modified catalyst system comprising two or more layers: a first catalyst layer comprising one or more modified mixed metal oxide catalysts and (b) at least a second catalyst layer comprising at least one unmodified or modified metal oxide, supported or unsupported, and is oriented downstream from the first catalyst layer; wherein the catalyst is enhanced in X and Z by vapor depositing at least element of X, Z or combinations thereof.

**6.** A surface modified catalyst system comprising two or more layers: a first catalyst layer comprising one or more modified mixed metal oxide catalysts, wherein the one or more modified mixed metal oxide catalysts is improved with a sub-monolayer deposition of Te onto its surface by vapor deposition; and (b) at least a second catalyst layer comprising at least one unmodified or modified metal oxide, supported or unsupported, and is oriented downstream from the first catalyst layer; wherein the catalyst surface is modified in X and Z by vapor depositing at least element of X, Z or combinations thereof on to the surface of the mixed metal oxide catalyst.

**7.** A process for enhancing, rebuilding, replenishing or reconstructing the surface of one or more mixed metal oxide catalysts comprising the step of: depositing one or more elements X and Z in the vapor phase, wherein X is at least one element selected from the group consisting of Te and Sb, Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements, to one or more metals to one or more mixed metal catalysts and wherein the one or more modified mixed metal oxide catalysts is improved with a sub-monolayer deposition of Te onto its surface by vapor deposition.

European Patent Office

# PARTIAL EUROPEAN SEARCH REPORT

which under Rule 45 of the European Patent Convention shall be considered, for the purposes of subsequent proceedings, as the European search report

Application Number
EP 05 25 7253

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 907 052 A (HAMADA ET AL) 25 May 1999 (1999-05-25) * claim 1; example 1 * | 1-4 | B01J23/00 B01J27/057 B01J37/02 C07C51/215 C07C57/04 C07C253/24 C07C255/08 |
| A | US 4 774 352 A (SASAKI ET AL) 27 September 1988 (1988-09-27) * column 2, line 8 - line 14 * * column 4, line 63 - line 66 * | | |
| A | US 4 618 593 A (SASAKI ET AL) 21 October 1986 (1986-10-21) * column 2, line 50 - line 55 * * column 5, line 55 - line 59 * | | |
| A | GAFFNEY A M ET AL: "Characterization and catalytic studies of PVD synthesized Mo/V/Nb/Te oxide catalysts" JOURNAL OF CATALYSIS, ACADEMIC PRESS, DULUTH, MN, US, vol. 229, no. 1, 12 November 2004 (2004-11-12), - 1 January 2005 (2005-01-01) pages 12-23, XP004693494 ISSN: 0021-9517 Available Online from 12-11-2004 | | TECHNICAL FIELDS SEARCHED (IPC): C07C B01J |

-/--

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

2

| Place of search | Date of completion of the search | Examiner |
|---|---|---|
| The Hague | 10 March 2006 | Veefkind, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

# PARTIAL EUROPEAN SEARCH REPORT

Application Number
EP 05 25 7253

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| A | US 2003/013904 A1 (CHATURVEDI SANJAY ET AL) 16 January 2003 (2003-01-16)<br>----- | | |
| A | EP 1 266 688 A (ROHM AND HAAS COMPANY) 18 December 2002 (2002-12-18)<br>----- | | |
| A | US 6 642 174 B2 (GAFFNEY A M) 4 November 2003 (2003-11-04)<br>----- | | |
| | | | TECHNICAL FIELDS SEARCHED (IPC) |

2

EPO FORM 1503 03.82 (P04C10)

European Patent Office

INCOMPLETE SEARCH SHEET C

Application Number
EP 05 25 7253

Claim(s) searched incompletely:
1-4

Claim(s) not searched:
5-7

Reason for the limitation of the search:

Claims 5 and 6 are extremely vague and unclear and lack support. The features X and Y are not defined. The terms "modified" or "unmodiefied" are also highly unclear and could mean anything. Even when looking at the description, for example at page 7 of the description, it appears that the only definition of "modified" appears to be that it is different from "unmodified"! (see page 7, lines 13-18 This, however, can be said to be the case for any catalyst preparation and does not impose any limitation whatsoever. This leads to a fundamental lack of clarity for these claims. Performing a meaningful Search for these claims is not possible.

It appears from the description that the only vapor phase deposition performed by the applicant, and for which some effect is claimed, is in fact that of Te, and this only in combination with specific mixed metal oxide catalysts. The subject-matter of the claims 3,4 and 7, where about half of the elements of the Periodic Table may be added through the vapor phase, goes well beyond the disclosure of the description and is considered to be unsupported (Article 84 EPC).

The only example for the effect of Te addition through the vapor phase deals with a very specific catalyst consisting of V, Mo, Nb and Te. None of the other elements, which are mentioned in claims 1-4 and 7 have any support (Article 84 EPC) in the form of examples.

Thus, drafting of the claims is such that a meaningful search over the whole claimed subject-matter is not possible. As a result, the search has been limited to claims 1-4, insofar relating to catalysts consisting of Mo, V, Nb and Te -oxides.

## ANNEX TO THE EUROPEAN SEARCH REPORT ON EUROPEAN PATENT APPLICATION NO. EP 05 25 7253

This annex lists the patent family members relating to the patent documents cited in the above-mentioned European search report.
The members are as contained in the European Patent Office EDP file on
The European Patent Office is in no way liable for these particulars which are merely given for the purpose of information.

10-03-2006

| Patent document cited in search report | Publication date | Patent family member(s) | Publication date |
|---|---|---|---|
| US 5907052 A | 25-05-1999 | CN 1220258 A | 23-06-1999 |
| | | DE 19836359 A1 | 04-03-1999 |
| | | HK 1018951 A1 | 14-03-2003 |
| | | ID 21268 A | 12-05-1999 |
| US 4774352 A | 27-09-1988 | DE 3274975 D1 | 12-02-1987 |
| | | EP 0076678 A2 | 13-04-1983 |
| US 4618593 A | 21-10-1986 | AT 384558 B | 10-12-1987 |
| | | AT 189882 A | 15-05-1987 |
| | | BG 50715 A3 | 15-10-1992 |
| | | BR 8202794 A | 26-04-1983 |
| | | CA 1189844 A1 | 02-07-1985 |
| | | DD 202630 A5 | 28-09-1983 |
| | | DD 210218 A5 | 06-06-1984 |
| | | DE 3217700 A1 | 02-12-1982 |
| | | ES 8306607 A1 | 16-09-1983 |
| | | ES 8403040 A1 | 01-06-1984 |
| | | FR 2505675 A1 | 19-11-1982 |
| | | GB 2101004 A | 12-01-1983 |
| | | GB 2163365 A | 26-02-1986 |
| | | IT 1154303 B | 21-01-1987 |
| | | KR 8903702 B1 | 30-09-1989 |
| | | MX 162726 A | 20-06-1991 |
| | | NL 8202012 A | 01-12-1982 |
| | | SU 1367844 A3 | 15-01-1988 |
| | | US 4709070 A | 24-11-1987 |
| | | US 4709071 A | 24-11-1987 |
| US 2003013904 A1 | 16-01-2003 | US 2004030202 A1 | 12-02-2004 |
| EP 1266688 A | 18-12-2002 | BR 0202256 A | 01-04-2003 |
| | | CN 1391984 A | 22-01-2003 |
| | | CN 1714934 A | 04-01-2006 |
| | | JP 2003053189 A | 25-02-2003 |
| | | TW 574071 B | 01-02-2004 |
| US 6642174 B2 | 04-11-2003 | US 2002188150 A1 | 12-12-2002 |
| | | US 2004044248 A1 | 04-03-2004 |

EPO FORM P0459

For more details about this annex : see Official Journal of the European Patent Office, No. 12/82